# EUROPEAN PATENT SPECIFICATION

(11) **EP 3 455 621 B1**
(45) Date of publication and mention of the grant of the patent: **19.07.2023**
(21) Application number: 17796999.5
(22) Date of filing: 12.05.2017
(51) Int. Cl.: C12Q 1/6883, G01N 33/68, C12Q 1/68

(54) **SIMPLIFICATION OF A SEPTIC SHOCK ENDOTYPING STRATEGY FOR CLINICAL APPLICATION**
VEREINFACHUNG EINER ENDOTYPING-STRATEGIE FÜR SEPTISCHEN SCHOCK ZUR KLINISCHEN ANWENDUNG
SIMPLIFICATION D'UNE STRATÉGIE D'ENDOTYPAGE DE CHOC SEPTIQUE POUR UNE APPLICATION CLINIQUE

(30) Priority: 13.05.2016 US 201662335803 P; 29.11.2016 US 201662427778 P; 30.11.2016 US 201662428451 P; 13.01.2017 US 201762446216 P
(43) Date of publication of application: 20.03.2019
(73) Proprietor: Cincinnati Children's Hospital Medical Center, Cincinnati, OH 45229-3039 (US); University of Cincinnati, Cincinnati, OH 45221-0623 (US)
(72) Inventor: WONG, Hector R., Cincinnati, Ohio 45208 (US); LINDSELL, Christopher John, Cincinnati, Ohio 45242 (US)
(74) Representative: Forresters IP LLP
(86) International application number: PCT/US2017/032538
(87) International publication number: WO 2017/197352

(56) References cited:
- US-A1- 2009 312 194
- US-A1- 2013 209 473
- US-A1- 2014 314 865
- US-A1- 2014 323 391
- US-A1- 2014 323 391
- US-A1- 2014 349 890
- US-A1- 2015 018 238
- WONG HECTOR R ET AL: "Identification of pediatric septic shock subclasses based on genome-wide expression profiling", BMC MEDICINE, BIOMED CENTRAL LTD., LONDON, GB, vol. 7, no. 1, 22 July 2009 (2009-07-22), page 34, XP021059282, ISSN: 1741-7015, DOI: 10.1186/1741-7015-7-34
- HECTOR R. WONG ET AL: "Developing a Clinically Feasible Personalized Medicine Approach to Pediatric Septic Shock", AMERICAN JOURNAL OF RESPIRATORY AND CRITICAL CARE MEDICINE., vol. 191, no. 3, 1 February 2015 (2015-02-01), pages 309-315, XP055641552, US ISSN: 1073-449X, DOI: 10.1164/rccm.201410-1864OC & Hector R. Wong ET AL: "Developing a Clinically Feasible Personalized Medicine Approach to Pediatric Septic Shock", AMERICAN JOURNAL OF RESPIRATORY AND CRITICAL CARE MEDICINE., vol. 191, no. 3, 1 February 2015 (2015-02-01), pages 309-315, XP055641556, US ISSN: 1073-449X, DOI: 10.1164/rccm.201410-1864OC
- GEISS GARY K ET AL: "Direct multiplexed measurement of gene expression with color-coded probe pairs", NATURE BIOTECHNOLOGY, GALE GROUP INC, NEW YORK, vol. 26, no. 3, 1 March 2008 (2008-03-01), pages 317-325, XP002505107, ISSN: 1087-0156, DOI: 10.1038/NBT1385
- STEPHEN W STANDAGE ET AL: "Biomarkers for pediatric sepsis and septic shock", EXPERT REVIEW OF ANTI-INFECTIVE THERAPY, vol. 9, no. 1, 1 January 2011 (2011-01-01) , pages 71-79, XP55161270, ISSN: 1478-7210, DOI: 10.1586/eri.10.154
- HECTOR R. WONG ET AL: "CORRESPONDENCE Simplification of a Septic Shock Endotyping Strategy for Clinical Application", AMERICAN JOURNAL OF RESPIRATORY AND CRITICAL CARE MEDICINE, vol. 195, no. 2, 15 January 2017 (2017-01-15), pages 263-265, XP055641570,

## Description

### FIELD OF THE INVENTION

The invention is defined in the claim. Embodiments and descriptions no longer falling under the scope of the appended claims are considered merely as examples suitable for understanding the invention. The disclosure herein generally relates to the identification and validation of clinically relevant, quantifiable biomarkers of diagnostic and therapeutic responses for blood, vascular, cardiac, and respiratory tract dysfunction.

### BACKGROUND

Septic shock and severe sepsis represent a major public health problem in the United States, despite the development of increasingly powerful antibiotics and advanced forms of intensive care unit-based support modalities (see, *e*.*g*., Shanley, T. et al. Sepsis, 3rd Ed., St. Louis, MO, Mosby (2006)). Worldwide, septic shock affects millions of adults, killing approximately one in four (see, *e.g.,* Dellinger, R. et al. Crit. Care Med. 36:296-327 (2008)). One study suggests that the incidence and the mortality rates of septic shock in adults are increasing in the United States (Dombrovskiy, V. et al. Crit. Care Med. 35:1244-50 (2007)).

Septic shock is also a major problem in the pediatric age group, as there are ~42,000 cases of pediatric septic shock per year in the United States alone, with a mortality rate of ~10% (see, *e.g.,* Watson, R. et al. Am. J. Respir. Crit. Care Med. 167:695-701 (2003)). While the pediatric mortality rate is lower than that of adults, it nonetheless translates to more than 4,000 childhood deaths per year and countless years of lost productivity due to death at a young age. While this high number of pediatric deaths per year from septic shock indicates that more children die per year in the United States from septic shock as the primary cause than those children who die from cancer, funding specifically targeted toward pediatric septic shock is substantially lower than that for pediatric cancer.

Septic shock is a clinical syndrome with substantial clinical and biological heterogeneity. As such, reliable stratification of outcome risk is fundamental to effective clinical practice and clinical research (Marshall J. Leukoc. Biol. 83:471-82 (2008)), as is the ability to differentiate between subclasses of septic shock that have different biology. For example, septic shock endotypes can have differences with respect to adaptive immunity and glucocorticoid receptor signaling, as well as different responses to steroid treatment. Risk stratification tools specific for septic shock in pediatric patients would be beneficial at several levels, including stratification for interventional clinical trials, better-informed decision making for individual patients (*i.e.* prognostication), and as a metric for quality improvement efforts. A recent Lancet Infectious Diseases Commission by Cohen *et al.* has outlined a roadmap for future research in the field of sepsis, including the identification of sepsis subclasses as a means of resolving heterogeneity and improving patient outcomes [1].

### SUMMARY OF THE INVENTION

The invention is defined in the claims. The invention relates to a method of classifying a pediatric patient with septic shock as high risk or low risk of adverse outcome, wherein the method includes analyzing a sample from a pediatric patient with septic shock to determine the expression levels of two or more biomarkers selected from the group consisting of JAK2, LYN, PRKCB, and SOS2; determining whether the expression levels of the two or more biomarkers are elevated above a cut-off level; and classifying the patient is classified as endotype A / high risk or other than endotype A / high risk, wherein a classification of endotype A / high risk includes: a) a non-elevated level of JAK2 and a non-elevated level of PRKCB; or b) a non-elevated level of JAK2, an elevated level of PRKCB, and a non-elevated level of LYN; and wherein a classification other than endotype A / high risk includes: c) a non-elevated level of JAK2, an elevated level of PRKCB, and an elevated level of LYN; or d) an elevated level of JAK2, a non-elevated level of SOS2, and a highly elevated level of LYN; or e) elevated levels of JAK2 and SOS2; or f) an elevated level of JAK2, a non-elevated level of SOS2, and a non-highly elevated level of LYN.

In certain embodiments of the methods, biomarker expression levels can be determined by mRNA quantification. In some embodiments, biomarker expression levels can be determined by normalized mRNA counts and/or by cycle threshold (CT) values.

In certain embodiments of the methods, the biomarkers include a pair of biomarkers selected from JAK2 and LYN; JAK2 and PRKCB; JAK2 and SOS2; LYN and PRKCB; LYN and SOS2; and PRKCB and SOS2. In some embodiments, the biomarkers include three or more selected from JAK2, LYN, PRKCB, and SOS2. In some embodiments, the biomarkers include a trio of biomarkers selected from JAK2, LYN, and PRKCB; JAK2, LYN, and SOS2; JAK2, PRKCB, and SOS2; LYN, PRKCB, and SOS2. In some embodiments, the biomarkers include all of JAK2, LYN, PRKCB, and SOS2.

In certain embodiments of the methods, biomarker levels can be determined by normalized mRNA counts, wherein: a) an elevated level of JAK2 corresponds to a serum JAK2 mRNA count greater than 1780; b) an elevated level of PRKCB corresponds to a serum PRKCB mRNA count greater than 990; c) an elevated level of SOS2 corresponds to a serum SOS2 mRNA count greater than 480; d) an elevated level of LYN corresponds to a serum LYN mRNA count greater than 870; and e) a highly elevated level of LYN corresponds to a serum LYN mRNA count greater than 940.

In certain embodiments of the methods, biomarker levels can be determined by normalized mRNA counts, wherein: a) an elevated level of JAK2 corresponds to a serum JAK2 mRNA count greater than 1784; b) an elevated level of PRKCB corresponds to a serum PRKCB mRNA count greater than 986; c) an elevated level of SOS2 corresponds to a serum SOS2 mRNA count greater than 480; d) an elevated level of LYN corresponds to a serum LYN mRNA count greater than 873; and e) a highly elevated level of LYN corresponds to a serum LYN mRNA count greater than 938.

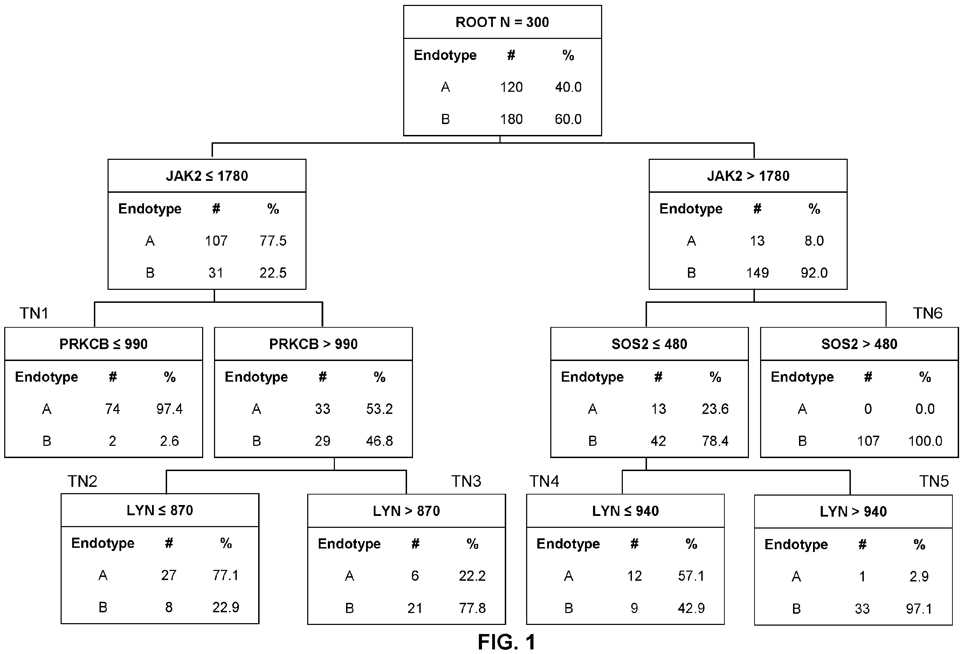

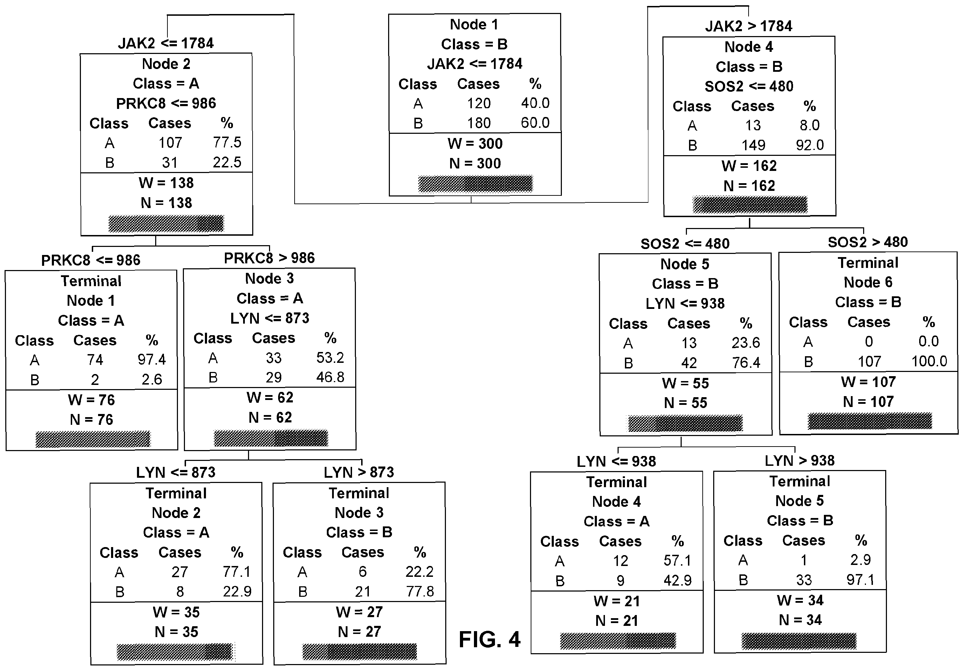

In certain embodiments of the methods, the determination of whether the levels of the two or more biomarkers are non-elevated above a cut-off level includes applying the biomarker expression level data to a decision tree including the two or more biomarkers. Some embodiments of the methods include application of the decision tree of Figure 1. Some embodiments of the methods include application of the decision tree of Figure 4.

In certain embodiments of the methods, a classification other than endotype A / high risk includes a classification of endotype B / low risk or endotype C / moderate risk.

In certain embodiments of the methods, the determination of whether the levels of the two or more biomarkers are non-elevated can be combined with one or more patient demographic data and/or clinical characteristics and/or results from other tests or indicia of septic shock. In some embodiments, the patient demographic data and/or clinical characteristics and/or results from other tests or indicia of septic shock include at least one of the septic shock causative organism, the presence or absence or chronic disease, and/or the age, gender, race, and/or co-morbidities of the patient. In some embodiments, the determination of whether the levels of the two or more biomarkers are non-elevated above a cut-off level can be combined with one or more additional population-based risk scores. In some embodiments, the one or more population-based risk scores includes at least one of Pediatric Risk of Mortality (PRISM), Pediatric Index of Mortality (PIM), and/or Pediatric Logistic Organ Dysfunction (PELOD).

In some embodiments, the sample can be obtained within the first hour of presentation with septic shock. In some embodiments, the sample can be obtained within the first 48 hours of presentation with septic shock.

Certain embodiments of the methods, not being part of the invention, further include administering a treatment including one or more corticosteroid to a patient that is not endotype A / high risk, or administering a treatment including one or more therapy excluding a corticosteroid to a patient that is classified as endotype A / high risk, to provide a method of treating a pediatric patient with septic shock. In some embodiments, not being part of the invention, one or more high risk therapy can be administered to a patient classified as endotype A / high risk. In some embodiments, the one or more high risk therapy includes at least one of immune enhancing therapy, extracorporeal membrane oxygenation/life support, plasmapheresis, pulmonary artery catheterization, and/or high volume continuous hemofiltration. In some embodiments, the immune enhancing therapy includes GMCSF, interleukin-7, and/or anti-PD-1. Some embodiments of the methods include improving an outcome in a pediatric patient with septic shock.

Certain embodiments of the methods further include analyzing the subsequent sample that has been obtained from the patient at a later time point, to determine the expression levels of the two or more biomarkers selected from the group consisting of JAK2, LYN, PRKCB and SOS2, and determining whether the expression levels of the two or more biomarkers are non-elevated above a cut-off level, thereby determining a change to the patient's endotype classification; and maintaining the treatment being administered if the patient's endotype classification has not changed, or changing the treatment being administered if the patient's endotype classification has changed.

The disclosure relates to methods of classifying a pediatric patient with septic shock, not being part of the invention, wherein the methods include analyzing a sample from a pediatric patient with septic shock to determine the expression levels of biomarkers including three or more members of a quartet selected from GNAI3, PIK3C3, TLR1, and TYROBP; CD247, ITGAX, RHOT1, and TLR1; ARPC5, CSNK1A1, FCGR2C, and PPP2R5C; ASAHI, FCGR2C, TRA, and TYROBP; BTK, FAS, MAP3K3, and PPP2R2A; CASP4, CD247, EP300, and MAP3K3; and CREB5, CSNK1A1, PRKCB, and TLR2; and determining whether the expression levels of the three or more biomarkers are elevated above a cut-off level.

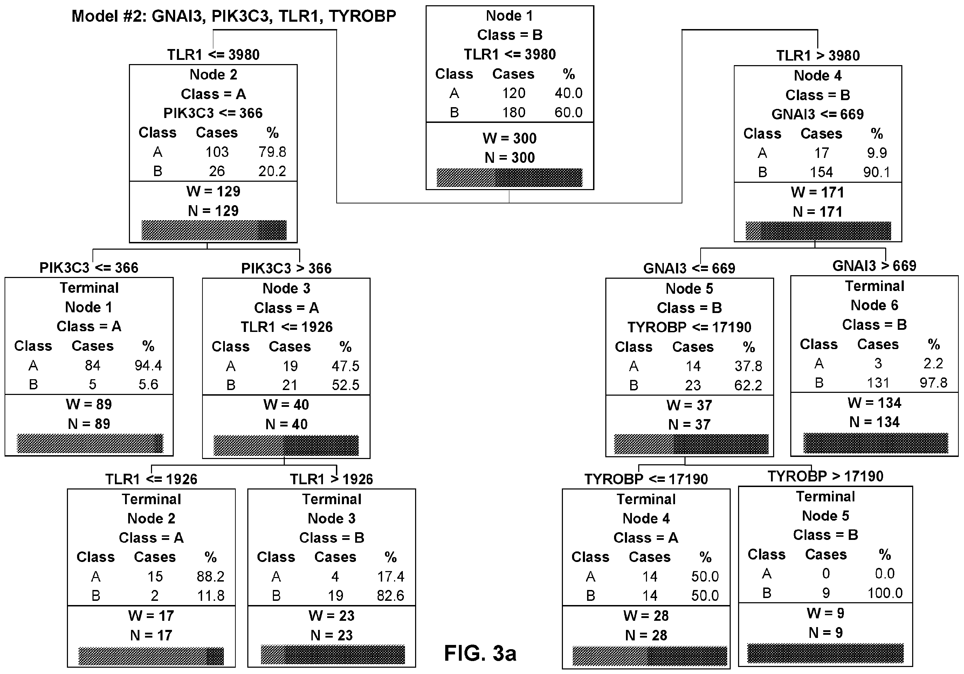

In certain embodiments of the methods, not being part of the invention, a classification of endotype A includes: a) a non-highly elevated level of TLR1 and a non-elevated level of PIK3C3; or b) a non-elevated level of TLR1, and an elevated level of PIK3C3; or c) a highly elevated level of TLR1, a non-elevated level of GNAI3, and a non-elevated level of TYROBP; and a classification other than endotype A / high risk includes: d) an elevated level of TLR1, and an elevated level of PIK3C3; or e) a highly elevated level of TLR1, a non-elevated level of GNAI3, and an elevated level of TYROBP; or f) a highly elevated level of TLR1, and an elevated level of GNAI3. In some embodiments, biomarker levels can be determined by normalized mRNA counts, wherein: a) an elevated level of TLR1 corresponds to a serum TLR1 mRNA count greater than 1926; b) a highly elevated level of TLR1 corresponds to a serum TLR1 mRNA count greater than 3980; c) an elevated level of PIK3C3 corresponds to a serum PIK3C3 mRNA count greater than 366; d) an elevated level of GNAI3 corresponds to a serum GNAI3 mRNA count greater than 669; and e) an elevated level of TYROBP corresponds to a serum TYROBP mRNA count greater than 17190. Some embodiments of the methods include application of the decision tree of Figure 3a.

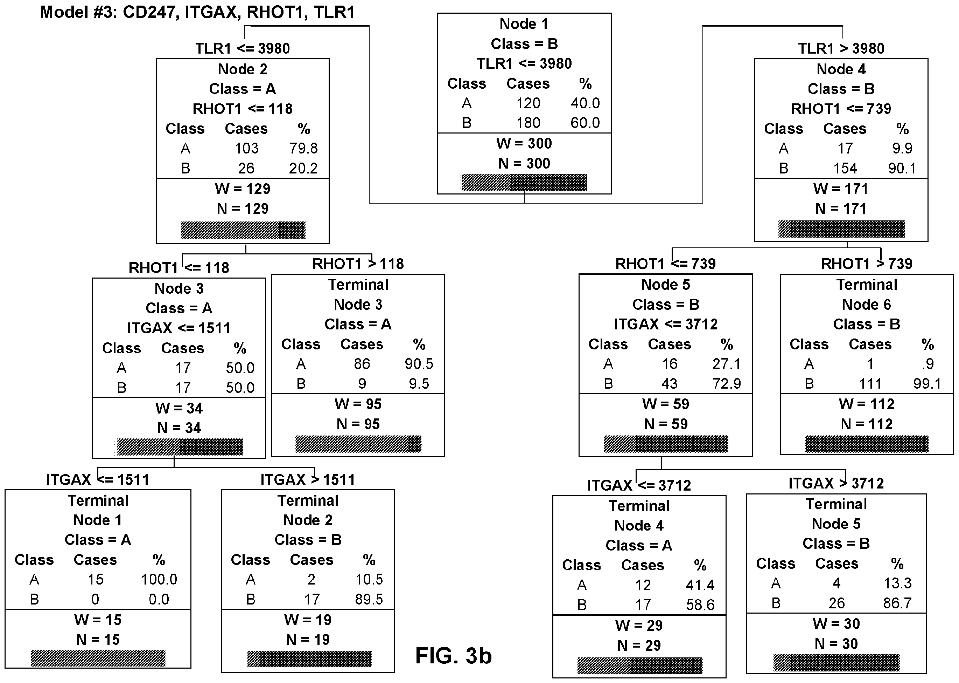

In certain embodiments of the methods, not being part of the invention, a classification of endotype A / high risk includes: a) a non-elevated level of TLR1, a non-elevated level of RHOT1, and a non-elevated level of ITGAX; or b) a non-elevated level of TLR1, and an elevated level of RHOT1; or c) an elevated level of TLR1, a non-highly elevated level of RHOT1, and a non-highly elevated level of ITGAX; and a classification other than endotype A / high risk includes: d) a non-elevated level of TLR1, a non-elevated level of RHOT1, and an elevated level of ITGAX; or e) an elevated level of TLR1, a non-highly elevated level of RHOT1, and a highly elevated level of ITGAX; or f) an elevated level of TLR1, and a highly elevated level of RHOT1. In some embodiments, biomarker levels can be determined by normalized mRNA counts, wherein: a) an elevated level of TLR1 corresponds to a serum TLR1 mRNA count greater than 3980; b) an elevated level of RHOT1 corresponds to a serum RHOT1 mRNA count greater than 118; c) a highly elevated level of RHOT1 corresponds to a serum RHOT1 mRNA count greater than 739; d) an elevated level of ITGAX corresponds to a serum ITGAX mRNA count greater than 1511; and e) a highly elevated level of ITGAX corresponds to a serum ITGAX mRNA count greater than 3712. Some embodiments of the methods include application of the decision tree of Figure 3b.

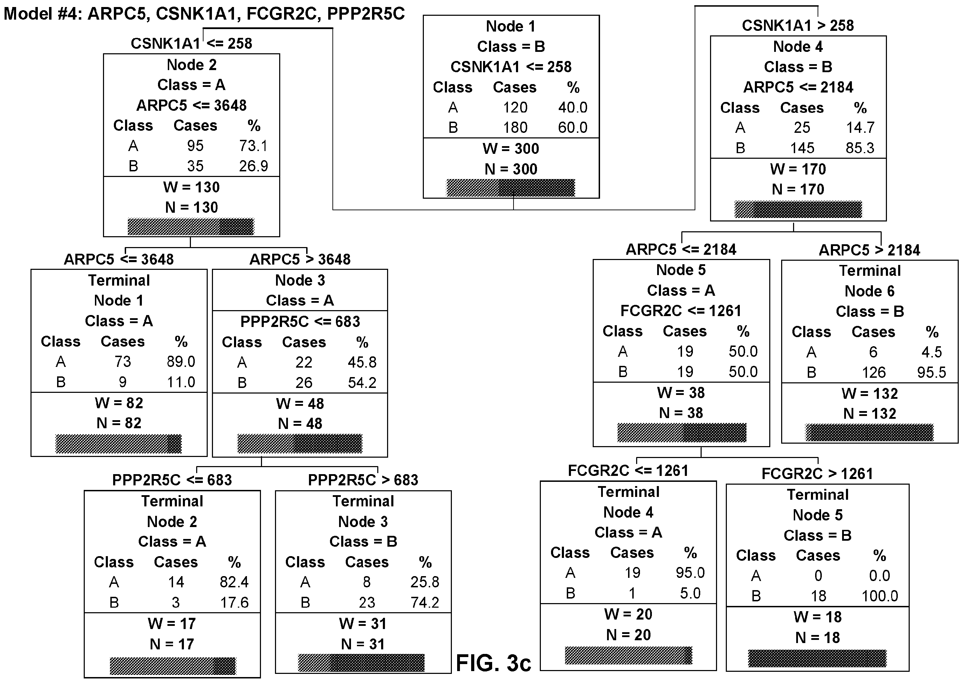

In certain embodiments of the methods, not being part of the invention, a classification of endotype A / high risk includes: a) a non-elevated level of CSNK1A1 and a non-highly elevated level of ARPC5; or b) a non-elevated level of CSNK1A1, a highly elevated level of ARPC5, and a non-elevated level of PPP2R5C; or c) an elevated level of CSNK1A1, a non-elevated level of ARPC5, and a non-elevated level of FCGR2C; and a classification other than endotype A / high risk includes: d) a non-elevated level of CSNK1A1, a highly elevated level of ARPC5, and an elevated level of PPP2R5C; or e) an elevated level of CSNK1A1, a non-elevated level of ARPC5, and an elevated level of FCGR2C; or f) an elevated level of CSNK1A1, and an elevated level of ARPC5. In some embodiments, biomarker levels can be determined by normalized mRNA counts, wherein: a) an elevated level of CSNK1A1 corresponds to a serum CSNK1A1 mRNA count greater than 258; b) an elevated level of ARPC5 corresponds to a serum ARPC5 mRNA count greater than 2184; c) a highly elevated level of ARPC5 corresponds to a serum ARPC5 mRNA count greater than 3648; d) an elevated level of PPP2R5C corresponds to a serum PPP2R5C mRNA count greater than 683; and e) an elevated level of FCGR2C corresponds to a serum FCGR2C mRNA count greater than 1261. Some embodiments of the methods include application of the decision tree of Figure 3c.

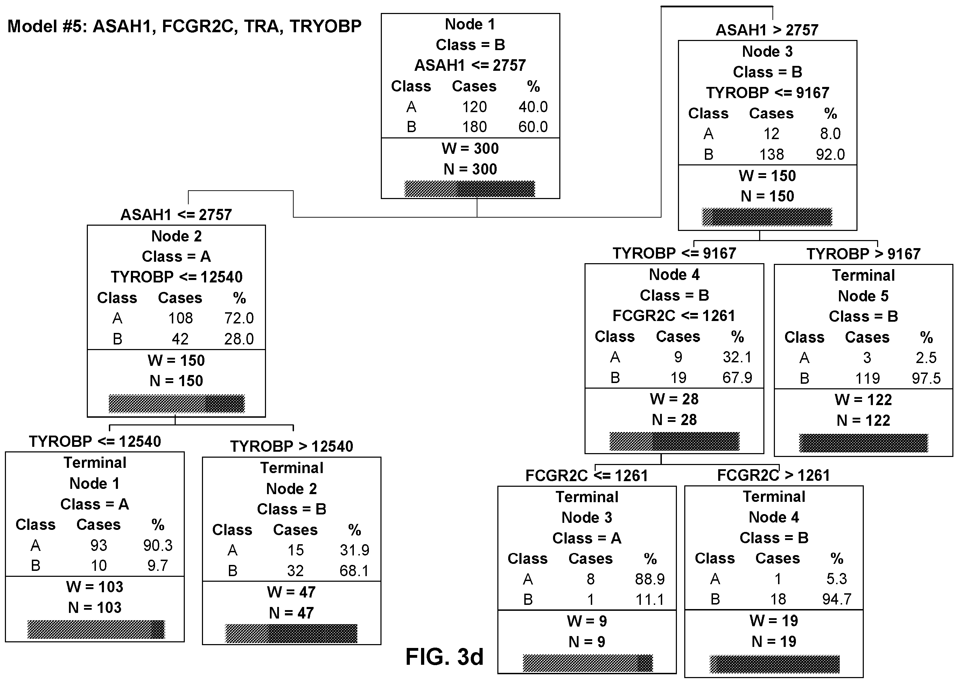

In certain embodiments of the methods, not being part of the invention, a classification of endotype A / high risk includes: a) a non-elevated level of ASAH1 and a non-highly elevated level of TYROBP; or b) an elevated level of ASAHI, a non-elevated level of TYROBP, and a non-elevated level of FCGR2C; and a classification other than endotype A / high risk includes: c) a non-elevated level of ASAH1 and a highly elevated level of TYROBP; or d) an elevated level of ASAHI, a non-elevated level of TYROBP, and an elevated level of FCGR2C; or e) an elevated level of ASAHI, and an elevated level of TYROBP. In some embodiments, biomarker levels can be determined by normalized mRNA counts, wherein: a) an elevated level of ASAH1 corresponds to a serum ASAH1 mRNA count greater than 2757; b) an elevated level of TYROBP corresponds to a serum TYROBP mRNA count greater than 9167; c) a highly elevated level of TYROBP corresponds to a serum TYROBP mRNA count greater than 12540; and d) an elevated level of FCGR2C corresponds to a serum FCGR2C mRNA count greater than 1261. Some embodiments of the methods include application of the decision tree of Figure 3d.

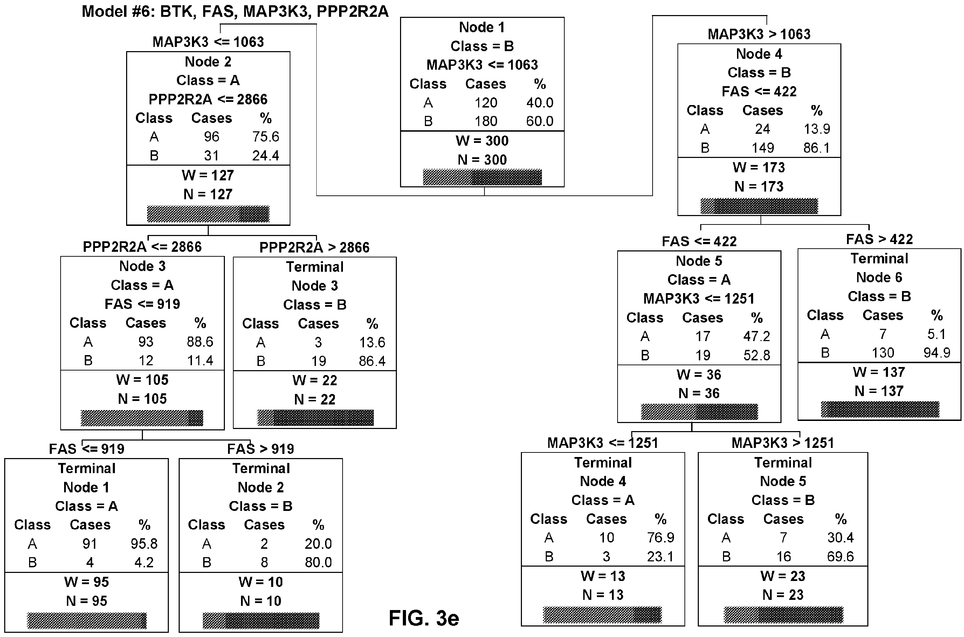

In certain embodiments of the methods, not being part of the invention, a classification of endotype A / high risk includes: a) a non-elevated level of MAP3K3, a non-elevated level of PPP2R2A, and a non-highly elevated level of FAS; or b) an elevated level of MAP3K3 which is not a highly elevated level of MAP3K3, and a non-elevated level of FAS; and a classification other than endotype A / high risk includes: c) a non-elevated level of MAP3K3, a non-elevated level of PPP2R2A, and a highly elevated level of FAS; or d) a non-elevated level of MAP3K3, and an elevated level of PPP2R2A; or e) a highly elevated level of MAP3K3, and a non-elevated level of FAS; or f) an elevated level of MAP3K3, and an elevated level of FAS. In some embodiments, biomarker levels can be determined by normalized mRNA counts, wherein: a) an elevated level of MAP3K3 corresponds to a serum MAP3K3 mRNA count greater than 1063; b) a highly elevated level of MAP3K3 corresponds to a serum MAP3K3 mRNA count greater than 1251; c) an elevated level of PPP2R2A corresponds to a serum PPP2R2A mRNA count greater than 2866; d) an elevated level of FAS corresponds to a serum FAS mRNA count greater than 422; and e) a highly elevated level of FAS corresponds to a serum FAS mRNA count greater than 919. Some embodiments of the methods include application of the decision tree of Figure 3e.

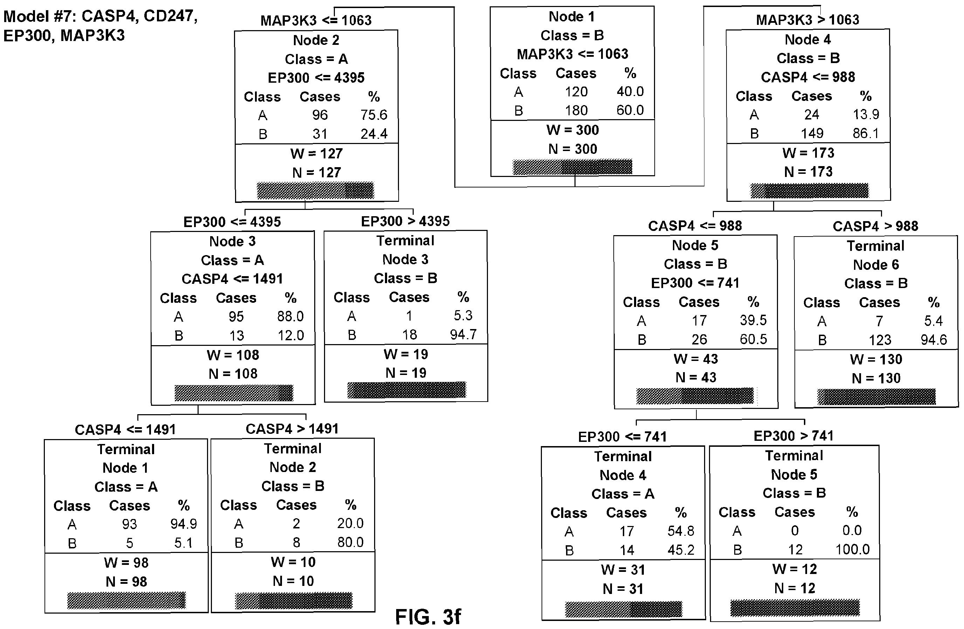

In certain embodiments of the methods, not being part of the invention, a classification of endotype A / high risk includes: a) a non-elevated level of MAP3K3, a non-highly elevated level of EP300, and a non-highly elevated level of CASP4; or b) an elevated level of MAP3K3, a non-elevated level of CASP4, and a non-elevated level of EP300; and a classification other than endotype A / high risk includes: c) a non-elevated level of MAP3K3, a non-highly elevated level of EP300, and a highly elevated level of CASP4; or d) a non-elevated level of MAP3K3, and a highly elevated level of EP300; or e) an elevated level of MAP3K3, a non-elevated level of CASP4, and a non-elevated level of EP300; or f) an elevated level of MAP3K3, and an elevated level of CASP4. In some embodiments, biomarker levels can be determined by normalized mRNA counts, wherein: a) an elevated level of MAP3K3 corresponds to a serum MAP3K3 mRNA count greater than 1063; b) an elevated level of EP300 corresponds to a serum EP300 mRNA count greater than 741; c) a highly elevated level of EP300 corresponds to a serum EP300 mRNA count greater than 4395; d) an elevated level of CASP4 corresponds to a serum CASP4 mRNA count greater than 988; and e) a highly elevated level of CASP4 corresponds to a serum CASP4 mRNA count greater than 1491. Some embodiments of the methods include application of the decision tree of Figure 3f.

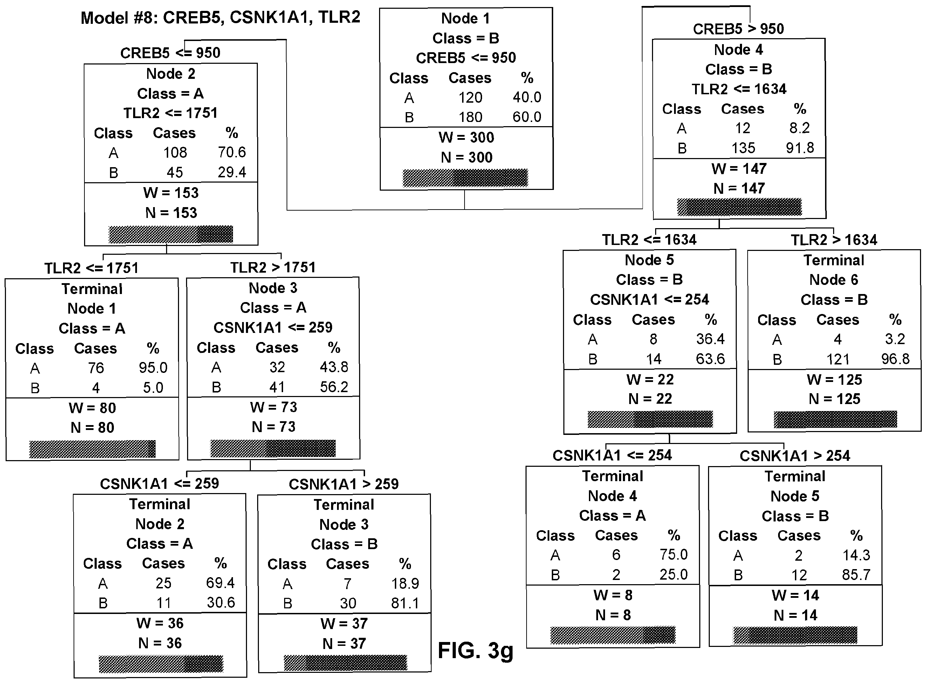

In certain embodiments of the methods, not being part of the invention, a classification of endotype A / high risk includes: a) a non-elevated level of CREB5, and a non-highly elevated level of TLR2; or b) a non-elevated level of CREB5, a highly elevated level of TLR2, and a non-highly elevated level of CSNK1A1; or c) an elevated level of CREB5, a non-elevated level of TLR2, and a non-elevated level of CSNK1A1; and a classification other than endotype A / high risk includes: d) a non-elevated level of CREB5, a highly elevated level of TLR2, and a highly elevated level of CSNK1A1; or e) an elevated level of CREB5, a non-elevated level of TLR2, and an elevated level of CSNK1A1; or f) an elevated level of CREB5, and an elevated level of TLR2. In some embodiments, biomarker levels can be determined by normalized mRNA counts, wherein: a) an elevated level of CREB5 corresponds to a serum CREB5 mRNA count greater than 950; b) an elevated level of TLR2 corresponds to a serum TLR2 mRNA count greater than 1634; c) a highly elevated level of TLR2 corresponds to a serum TLR2 mRNA count greater than 1751; d) an elevated level of CSNK1A1 corresponds to a serum CSNK1A1 mRNA count greater than 254; and e) a highly elevated level of CSNK1A1 corresponds to a serum CSNK1A1 mRNA count greater than 259. Some embodiments of the methods include application of the decision tree of Figure 3g.

The invention relates to the use of a diagnostic kit, test or array comprising a reporter hybridization probe, and a capture hybridization probe specific for each of two or more mRNA markers selected from the group consisting of JAK2, LYN, PRKCB, and SOS2 in a method according to claim 1. Certain embodiments further include a collection cartridge for immobilization of the hybridization probes. In some embodiments, the reporter and the capture hybridization probes include signal and barcode elements, respectively.

Certain disclosures ,not being part of the invention, relate to apparatuses or processing devices suitable for detecting two or more biomarkers selected from the biomarkers listed in Table 1. In certain embodiments, the biomarkers include three or more selected from ARPC5, ASAH1, BTK, CASP4, CD247, CREB5, CSNK1A1, EP300, FAS, FCGR2C, GNAI3, ITGAX, JAK2, LYN, MAP3K3, PIK3C3, PPP2R2A, PPP2R5C, PRKCB, RHOT1, SOS2, TLR1, TLR2, TRA, and TYROBP. In certain embodiments, the biomarkers include JAK2, LYN, PRKCB, and SOS2.

Certain disclosures ,not being part of the invention, relate to compositions including a reporter hybridization probe, and a capture hybridization probe specific for each of two or more mRNA markers selected from the biomarkers listed in Table 1. In certain embodiments, the mRNA markers include three or more selected from ARPC5, ASAH1, BTK, CASP4, CD247, CREB5, CSNK1A1, EP300, FAS, FCGR2C, GNAI3, ITGAX, JAK2, LYN, MAP3K3, PIK3C3, PPP2R2A, PPP2R5C, PRKCB, RHOT1, SOS2, TLR1, TLR2, TRA, and TYROBP. In certain embodiments, the mRNA markers include JAK2, LYN, PRKCB, and SOS2.

### BRIEF DESCRIPTION OF THE DRAWINGS

Those of skill in the art will understand that the drawings, described below, are for illustrative purposes only. The drawings are not intended to limit the scope of the present teachings in any way.
Figure 1 depicts decision tree #1, derived from model #1, including the genes *JAK1, PRKCB, SOS2,* and *LYN.* The gene expression values are provided in arbitrary units of mRNA counts, as generated by the NanoString nCounter platform and normalized to four housekeeping genes.
Figure 2 depicts the gene network involving the four classifier genes of model #1 generated by uploading the four classifier genes to the IPA platform. Gray shading highlights the network nodes corresponding to the four classifier genes, *JAK1, PRKCB, SOS2,* and *LYN.*
Figure 3 depicts decision trees #2-8, derived from models #2-8. Decision tree #2 (Figure 3a), derived from model #2, includes the genes *GNAI3, PIK3C3, TLR1,* and *TYROBP;* decision tree #3 (Figure 3b), derived from model #3, includes the genes *CD247, ITGAX, RHOT1,* and *TLR1;* decision tree #4 (Figure 3c), derived from model #4. includes the genes *ARPC5, CSNK1A1, FCGR2C,* and *PPP2RSC;* decision tree #5 (Figure 3d), derived from model #5, includes the genes *ASAH1, FCGR2C, TRA,* and *TYROBP;* decision tree #6 (Figure 3e), derived from model #6, includes the genes *BTK, FAS, MAP3K3,* and *PPP2R2A;* decision tree #7 (Figure 3f), derived from model #7, includes the genes *CASP4, CD247, EP300,* and *MAP3K3;* and decision tree #8 (Figure 3g), derived from model #8, includes the genes *CREB5, CSNK1A1,* and *TLR2.*
Figure 4 depicts an alternative version of decision tree #1, which was derived from using the 25 unique genes that appear in models #1 through #8 as candidate predictor variables to determine which of the genes "best" differentiate between endotypes A and B.

### DETAILED DESCRIPTION OF THE INVENTION

References cited herein include: United States Patent Application No. 61/595,996, BIOMARKERS OF SEPTIC SHOCK, filed on February 7, 2012; U.S. Provisional Application No. 61/721,705, A MULTI-BIOMARKER-BASED OUTCOME RISK STRATIFICATION MODEL FOR ADULT SEPTIC SHOCK, filed on November 2, 2012; International Patent Application No. PCT/US13/25223, A MULTI-BIOMARKER-BASED OUTCOME RISK STRATIFICATION MODEL FOR PEDIATRIC SEPTIC SHOCK, filed on February 7, 2013; International Patent Application No. PCT/US13/25221, A MULTI-BIOMARKER-BASED OUTCOME RISK STRATIFICATION MODEL FOR ADULT SEPTIC SHOCK, filed on February 7, 2013; U.S. Provisional Application No. 61/908,613, TEMPORAL PEDIATRIC SEPSIS BIOMARKER RISK MODEL, filed on November 25, 2013; and International Patent Application No. PCT/US14/067438, TEMPORAL PEDIATRIC SEPSIS BIOMARKER RISK MODEL, filed on November 25, 2014.

Unless otherwise noted, terms are to be understood according to conventional usage by those of ordinary skill in the relevant art.

As used herein, the term "sample" encompasses a sample obtained from a subject or patient. The sample can be of any biological tissue or fluid. Such samples include, but are not limited to, sputum, saliva, buccal sample, oral sample, blood, serum, mucus, plasma, urine, blood cells (*e*.*g*., white cells), circulating cells (*e.g.* stem cells or endothelial cells in the blood), tissue, core or fine needle biopsy samples, cell-containing body fluids, free floating nucleic acids, urine, stool, peritoneal fluid, and pleural fluid, tear fluid, or cells therefrom. Samples can also include sections of tissues such as frozen or fixed sections taken for histological purposes or microdissected cells or extracellular parts thereof. A sample to be analyzed can be tissue material from a tissue biopsy obtained by aspiration or punch, excision or by any other surgical method leading to biopsy or resected cellular material. Such a sample can comprise cells obtained from a subject or patient. In some embodiments, the sample is a body fluid that include, for example, blood fluids, serum, mucus, plasma, lymph, ascitic fluids, gynecological fluids, or urine but not limited to these fluids. In some embodiments, the sample can be a non-invasive sample, such as, for example, a saline swish, a buccal scrape, a buccal swab, and the like.

As used herein, "blood" can include, for example, plasma, serum, whole blood, blood lysates, and the like.

As used herein, the term "assessing" includes any form of measurement, and includes determining if an element is present or not. The terms "determining," "measuring," "evaluating," "assessing" and "assaying" can be used interchangeably and can include quantitative and/or qualitative determinations.

As used herein, the term "monitoring" with reference to septic shock refers to a method or process of determining the severity or degree of septic shock or stratifying septic shock based on risk and/or probability of mortality. In some embodiments, monitoring relates to a method or process of determining the therapeutic efficacy of a treatment being administered to a patient.

As used herein, "outcome" can refer to an outcome studied. In some embodiments, "outcome" can refer to 28-day survival / mortality. The importance of survival / mortality in the context of pediatric septic shock is readily evident. The common choice of 28 days was based on the fact that 28-day mortality is a standard primary endpoint for interventional clinical trials involving critically ill patients. In some embodiments, an increased risk for a poor outcome indicates that a therapy has had a poor efficacy, and a reduced risk for a poor outcome indicates that a therapy has had a good efficacy.

As used herein, "outcome" can also refer to resolution of organ failure after 14 days or 28 days or limb loss. Although mortality / survival is obviously an important outcome, survivors have clinically relevant short- and long-term morbidities that impact quality of life, which are not captured by the dichotomy of "alive" or "dead." In the absence of a formal, validated quality of life measurement tool for survivors of pediatric septic shock, resolution of organ failure can be used as a secondary outcome measure. For example, the presence or absence of new organ failure over one or more timeframes can be tracked. Patients having organ failure beyond 28 days are likely to survive with significant morbidities having negative consequences for quality of life. Organ failure is generally defined based on published and well-accepted criteria for the pediatric population (Goldstein, B. et al. Pediatr. Crit. Care Med. 6:208 (2005)). Specifically, cardiovascular, respiratory, renal, hepatic, hematologic, and neurologic failure can be tracked. In addition, limb loss can be tracked as a secondary outcome. Although limb loss is not a true "organ failure," it is an important consequence of pediatric septic shock with obvious impact on quality of life.

As used herein, "outcome" can also refer to complicated course. Complicated course as defined herein relates to persistence of two or more organ failures at day seven of septic shock or 28-day mortality.

As used herein, the terms "predicting outcome" and "outcome risk stratification" with reference to septic shock refers to a method or process of prognosticating a patient's risk of a certain outcome. In some embodiments, predicting an outcome relates to monitoring the therapeutic efficacy of a treatment being administered to a patient. In some embodiments, predicting an outcome relates to determining a relative risk of an adverse outcome (e.g. complicated course) and/or mortality when administered a particular treatment or treatment regimen. Such adverse outcome risk can be high risk, moderate risk, moderate-high risk, moderate-low risk, or low risk. Alternatively, such adverse outcome risk can be described simply as high risk or low risk, corresponding to high risk of adverse outcome (e.g. complicated course) and/or mortality, or high likelihood of therapeutic effectiveness, respectively. In some embodiments of the present invention, adverse outcome risk can be determined via the biomarker-based endotyping strategy described herein. In some embodiments, predicting an outcome relates to determining a relative risk of mortality. Such mortality risk can be high risk, moderate risk, moderate-high risk, moderate-low risk, or low risk. Alternatively, such mortality risk can be described simply as high risk or low risk, corresponding to high risk of death or high likelihood of survival, respectively. As related to the terminal nodes of the decision trees described herein, a "high risk terminal node" corresponds to an increased probability of adverse outcome (e.g. complicated course) and/or mortality according to a particular treatment or treatment regimen, whereas a "low risk terminal node" corresponds to a decreased probability of adverse outcome (e.g. complicated course) and/or mortality according to a particular treatment or treatment regimen.

As used herein, the term "high risk clinical trial" refers to one in which the test agent has "more than minimal risk" (as defined by the terminology used by institutional review boards, or IRBs). In some embodiments, a high risk clinical trial is a drug trial.

As used herein, the term "low risk clinical trial" refers to one in which the test agent has "minimal risk" (as defined by the terminology used by IRBs). In some embodiments, a low risk clinical trial is one that is not a drug trial. In some embodiments, a low risk clinical trial is one that that involves the use of a monitor or clinical practice process. In some embodiments, a low risk clinical trial is an observational clinical trial.

As used herein, the terms "modulated" or "modulation," or "regulated" or "regulation" and "differentially regulated" can refer to both up regulation (*i*.*e*., activation or stimulation, *e.g.,* by agonizing or potentiating) and down regulation (*i*.*e*., inhibition or suppression, *e*.*g*., by antagonizing, decreasing or inhibiting), unless otherwise specified or clear from the context of a specific usage.

As used herein, the term "subject" refers to any member of the animal kingdom. In some embodiments, a subject is a human patient. In some embodiments, a subject is a pediatric patient. In some embodiments, a pediatric patient is a patient under 18 years of age, while an adult patient is 18 or older.

As used herein, the terms "treatment," "treating," "treat," and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment," as used herein, covers any treatment of a disease in a subject, particularly in a human, and includes: (a) preventing the disease from occurring in a subject which may be predisposed to the disease but has not yet been diagnosed as having it; (b) inhibiting the disease, *i*.*e*., arresting its development; and (c) relieving the disease, *i*.*e*., causing regression of the disease and/or relieving one or more disease symptoms. "Treatment" can also encompass delivery of an agent or administration of a therapy in order to provide for a pharmacologic effect, even in the absence of a disease or condition.

As used herein, the term "marker" or "biomarker" refers to a biological molecule, such as, for example, a nucleic acid, peptide, protein, hormone, and the like, whose presence or concentration can be detected and correlated with a known condition, such as a disease state. It can also be used to refer to a differentially expressed gene whose expression pattern can be utilized as part of a predictive, prognostic or diagnostic process in healthy conditions or a disease state, or which, alternatively, can be used in methods for identifying a useful treatment or prevention therapy.

As used herein, the term "expression levels" refers, for example, to a determined level of biomarker expression. The term "pattern of expression levels" refers to a determined level of biomarker expression compared either to a reference (*e.g.* a housekeeping gene or inversely regulated genes, or other reference biomarker) or to a computed average expression value (*e.g.* in DNA-chip analyses). A pattern is not limited to the comparison of two biomarkers but is more related to multiple comparisons of biomarkers to reference biomarkers or samples. A certain "pattern of expression levels" can also result and be determined by comparison and measurement of several biomarkers as disclosed herein and display the relative abundance of these transcripts to each other.

As used herein, a "reference pattern of expression levels" refers to any pattern of expression levels that can be used for the comparison to another pattern of expression levels. In some disclosures, a reference pattern of expression levels is, for example, an average pattern of expression levels observed in a group of healthy or diseased individuals, serving as a reference group.

As used herein, the term "decision tree" refers to a standard machine learning technique for multivariate data analysis and classification. Decision trees can be used to derive easily interpretable and intuitive rules for decision support systems.

The intrinsic heterogeneity of septic shock implies the existence of distinct subgroups that can be classified by pathobiological mechanism or treatment response. Such subgroups are known as endotypes.

The researchers of the present disclosure have previously identified two subclasses of pediatric septic shock based on genome-wide expression profiling [2]. Subsequently, a classification system was derived based on computer-assisted image analysis of gene expression mosaics representing 100 genes [3-5]. The 100-gene signature is provided in Table 1 below.

**Table 1. List of 100 septic shock subclass-defining genes**

| **Gene Symbol** | **Genbank** | **Description** |
|---|---|---|
| APAF1 | NM_013229 | apoptotic peptidase activating factor 1 |
| ARPC5 | AL516350 | actin related protein 2/3 complex, subunit 5, 16kDa |
| ASAH1 | BC016828 | N-acylsphingosine amidohydrolase (acid ceramidase) 1 |
| ATP2B2 | U15688 | ATPase, Ca++ transporting, plasma membrane 2 |
| BCL6 | NM_001706 | B-cell CLL/lymphoma 6 |
| BMPR2 | AL046696 | bone morphogenetic protein receptor, type II (serine/threonine kinase) |
| BTK | NM_000061 | Bruton agammaglobulinemia tyrosine kinase |
| CAMK2D | AA777512 | calcium/calmodulin-dependent protein kinase (CaM kinase) II delta |
| CAMK2G | AA284757 | calcium/calmodulin-dependent protein kinase (CaM kinase) II gamma |
| CAMK4 | AL529104 | calcium/calmodulin-dependent protein kinase IV |
| CASP1 | AI719655 | caspase 1, apoptosis-related cysteine peptidase (interleukin 1, beta, convertase) |
| CASP2 | AU153405 | caspase 2, apoptosis-related cysteine peptidase |
| CASP4 | U25804 | caspase 4, apoptosis-related cysteine peptidase |
| CASP8 | BF439983 | caspase 8, apoptosis-related cysteine peptidase |
| CD247 | J04132 | CD247 molecule |
| CD3E | NM_000733 | CD3e molecule, epsilon (CD3-TCR complex) |
| CD3G | NM_000073 | CD3g molecule, gamma (CD3-TCR complex) |
| CD79A | M74721 | CD79a molecule, immunoglobulin-associated alpha |
| CREB1 | NM_004379 | cAMP responsive element binding protein 1 |
| CREB5 | NM_004904 | cAMP responsive element binding protein 5 |
| CSNK1A1 | AV704610 | Casein kinase 1, alpha 1 |
| CTNNB1 | AF130085 | catenin (cadherin-associated protein), beta 1, 88kDa |
| DAPP 1 | NM 014395 | dual adaptor of phosphotyrosine and 3-phosphoinositides |
| DBT | AI632010 | dihydrolipoamide branched chain transacylase E2 |
| EP300 | AI459462 | E1A binding protein p300 |
| FAS | X83493 | Fas (TNF receptor superfamily, member 6) |
| FCGR2A | NM_021642 | Fc fragment of IgG, low affinity IIa, receptor (CD32) |
| FCGR2C | U90939 | Fc fragment of IgG, low affinity IIc, receptor for (CD32) |
| FYN | S74774 | FYN oncogene related to SRC, FGR, YES |
| GK | NM_000167 | glycerol kinase |
| GNAI3 | J03005 | guanine nucleotide binding protein (G protein), alpha inhibiting activity polypeptide 3 |
| HDAC4 | NM_006037 | histone deacetylase 4 |
| HLA-DMA | X76775 | major histocompatibility complex, class II, DM alpha |
| HLA-DOA | AL581873 | major histocompatibility complex, class II, DO alpha |
| ICAM3 | NM_002162 | intercellular adhesion molecule 3 |
| IL1A | NM_000575 | interleukin 1, alpha |
| INPP5D | BC027960 | inositol polyphosphate-5-phosphatase, 145kDa |
| ITGAM | NM_000632 | integrin, alpha M (complement component 3 receptor 3 subunit) |
| ITGAV | AI093579 | integrin, alpha V (vitronectin receptor, alpha polypeptide, antigen CD51) |
| ITGAX | M81695 | integrin, alpha X (complement component 3 receptor 4 subunit) |
| JAK1 | AL039831 | Janus kinase 1 (a protein tyrosine kinase) |
| JAK2 | NM_004972 | Janus kinase 2 (a protein tyrosine kinase) |
| KAT2B | AV735100 | K(lysine) acetyltransferase 2B |
| LAT2 | AF257135 | linker for activation of T cells family, member 2 |
| LYN | AI356412 | v-yes-1 Yamaguchi sarcoma viral related oncogene homolog |
| MAP2K4 | NM_003010 | mitogen-activated protein kinase kinase 4 |
| MAP3K 1 | AA541479 | mitogen-activated protein kinase kinase kinase 1 |
| MAP3K3 | BG231756 | mitogen-activated protein kinase kinase kinase 3 |
| MAP3K5 | NM_005923 | mitogen-activated protein kinase kinase kinase 5 |
| MAP3K7 | NM_003188 | mitogen-activated protein kinase kinase kinase 7 |
| MAP4K1 | BE646618 | mitogen-activated protein kinase kinase kinase kinase 1 |
| MAP4K4 | AL561281 | mitogen-activated protein kinase kinase kinase kinase 4 |
| MAPK1 | AA129773 | mitogen-activated protein kinase 1 |
| MAPK 14 | AF100544 | mitogen-activated protein kinase 14 |
| MDH1 | AW952547 | Malate dehydrogenase 1, NAD (soluble) |
| MKNK1 | BC002755 | MAP kinase interacting serine/threonine kinase 1 |
| NCOA2 | AU145806 | Nuclear receptor coactivator 2 |
| NCR3 | AF031136 | natural cytotoxicity triggering receptor 3 |
| NFATC1 | NM_006162 | nuclear factor of activated T-cells, cytoplasmic, calcineurin-dependent 1 |
| PAK2 | AA287921 | p21 protein (Cdc42/Rac)-activated kinase 2 |
| PDPR | BE644918 | pyruvate dehydrogenase phosphatase regulatory subunit |
| PIAS 1 | NM_016166 | protein inhibitor of activated STAT, 1 |
| PIK3C2A | AA579047 | Phosphoinositide-3-kinase, class 2, alpha polypeptide |
| PIK3 C3 | NM_002647 | phosphoinositide-3-kinase, class 3 |
| PIK3CA | AA767763 | Phosphoinositide-3-kinase, catalytic, alpha polypeptide |
| PIK3CD | U86453 | phosphoinositide-3-kinase, catalytic, delta polypeptide |
| PIK3R1 | AI679268 | phosphoinositide-3-kinase, regulatory subunit 1 (alpha) |
| PLCG1 | AL022394 | phospholipase C, gamma 1 |
| POU2F2 | AA805754 | POU class 2 homeobox 2 |
| PPP1R12A | AI817061 | protein phosphatase 1, regulatory (inhibitor) subunit 12A |
| PPP2R2A | AI934447 | protein phosphatase 2 (formerly 2A), regulatory subunit B, alpha isoform |
| PPP2R5C | AL834350 | protein phosphatase 2, regulatory subunit B', gamma isoform |
| PRKAR1A | AI682905 | protein kinase, cAMP-dependent, regulatory, type I, alpha (tissue specific extinguisher 1) |
| PRKCB | M13975 | protein kinase C, beta |
| PSMB7 | AI248671 | Proteasome (prosome, macropain) subunit, beta type, 7 |
| PTEN | BC005821 | phosphatase and tensin homolog |
| PTPRC | NM_002838 | protein tyrosine phosphatase, receptor type, C |
| RAF1 | BI496583 | V-raf-1 murine leukemia viral oncogene homolog 1 |
| RHOT 1 | NM_018307 | ras homolog gene family, member T1 |
| ROCK1 | N22548 | Rho-associated, coiled-coil containing protein kinase 1 |
| SEMA4F | AF119878 | sema domain, immunoglobulin domain (Ig), transmembrane domain (TM) and short cytoplasmic domain, (semaphorin) 4F |
| SEMA6B | NM_020241 | sema domain, transmembrane domain (TM), and cytoplasmic domain, (semaphorin) 6B |
| SMAD4 | AL832789 | SMAD family member 4 |
| SOS1 | AW241962 | son of sevenless homolog 1 (Drosophila) |
| SOS2 | L20686 | son of sevenless homolog 2 (Drosophila) |
| SP1 | BG431266 | Sp1 transcription factor |
| TAF11 | BQ709323 | TAF11 RNA polymerase II, TATA box binding protein (TBP)-associated factor, 28kDa |
| TBK1 | NM_013254 | TANK-binding kinase 1 |
| TGFBR1 | AV700621 | Transforming growth factor, beta receptor 1 |
| TLE4 | AL358975 | transducin-like enhancer of split 4 (E(sp1) homolog, Drosophila) |
| TLR1 | AL050262 | toll-like receptor 1 |
| TLR2 | NM_003264 | toll-like receptor 2 |
| TLR8 | AW872374 | toll-like receptor 8 |
| TNFSF10 | AW474434 | tumor necrosis factor (ligand) superfamily, member 10 |
| TRA@ | L34703 | T cell receptor alpha locus |
| TYROBP | NM_003332 | TYRO protein tyrosine kinase binding protein |
| UBE3A | AF037219 | Ubiquitin protein ligase E3A |
| USP48 | NM_018391 | ubiquitin specific peptidase 48 |
| ZAP70 | AI817942 | zeta-chain (TCR) associated protein kinase 70kDa |
| ZDHHC17 | AI621223 | zinc finger, DHHC-type containing 17 |

The subclasses have important differences with respect to mortality, organ failure burden, and treatment response [5, 6]. Given these differences, and because the classifier genes reflect adaptive immunity and the glucocorticoid receptor signaling pathway, these subclasses can represent endotypes of pediatric septic shock which differ with respect to outcome and treatment response. Assigning patients to an endotype can therefore enable precision critical care medicine.

Identification of these endotypes can identify patients who are more likely to respond and/or who are candidates for to a certain type of treatment, such as, for example, immune enhancing therapies or corticosteroids, and the like. As described herein, the present researchers have demonstrated that prescription of corticosteroids was independently associated with increased odds of mortality in one of the two endotypes [5]. In a separate analysis, endotype assignment was combined with risk stratification to identify a subgroup of patients in whom prescription of corticosteroids was associated with improved outcomes [6].

Endotype assignment based on computer-assisted image analysis of a 100-gene expression signature is theoretically achievable, but is currently impractical and cannot be used in the context of critically ill patients with septic shock for whom decision-making is highly time-sensitive [7]. There is therefore a need for an effective endotyping strategy based on a much smaller number of genes, which can readily translate into a clinical test amenable to decision making in this patient population. In order to accomplish such an effective endotyping strategy, the existing 100 endotype-defining genes need to be reduced into a much smaller subset needed to accurately differentiate endotypes, with the subset preferably containing a minimum number of genes needed to accurately differentiate endotypes. However, such an endotyping strategy based on a smaller number of genes does not currently exist and has heretofore not been readily elucidated, given the high level of biological complexity.

As described herein, Classification and Regression Tree (CART) methodology was used to reduce the existing 100 endotype-defining genes into very small subsets of four genes, which can accurately differentiate endotypes in order to derive a simplified and clinically applicable biomarker-based endotyping strategy. Ten-fold cross validation was used to assess model fit. Via this CART-based approach, the septic shock endotyping strategy was successfully reduced to decision trees consisting of just four genes.

For example, decision tree #1, derived from model #1 (consisting of the four genes JAK, LYN, PRKCB, and SOS2) as described herein and shown in Figure 1, had an area under the receiver operating curve (AUROC) of 0.97 (95% CI: 0.95 to 0.99). The diagnostic test characteristics of decision tree #1 included a positive likelihood ratio of 8.9 (95% CI: 5.8 to 13.7) and a negative likelihood ratio of 0.07 (95% CI: 0.03 to 0.13) for identifying endotype A., leading to excellent test characteristics for distinguishing endotype A from endotype B. Allocation to endotype A was associated with increased odds of poor outcome, and corticosteroid prescription was associated with increased odds of mortality among endotype A subjects, but not endotype B subjects. These associations were evident after adjusting for illness severity and age, and are consistent with previous observations [5]. The four genes in decision tree #1 correspond to signaling pathways and a gene network relevant to septic shock biology.

While the four-gene model in decision tree #1 leads to reclassification of some patients, the identical phenotypes listed above were observed when the derivation and test cohort subjects were combined, despite the reclassifications. Accordingly, the four-gene decision tree #1, consisting of JAK, LYN, PRKCB, and SOS2, fully reproduces the three phenotypic features seen when classifying patients using all 100 genes. This result is biologically important and fundamental for the concept of endotyping.

These data indicate that the septic shock endotyping strategy was successfully reduced to a decision tree consisting of just four genes. The decision tree has excellent test characteristics for distinguishing endotype A from endotype B in both the derivation and test cohorts, although there were some reclassifications of subjects relative to the original, reference criterion endotyping strategy. Despite these reclassifications, allocation to endotype A remained independently associated with increased odds of poor outcome, and corticosteroid prescription remained independently associated with increased odds of mortality among endotype A subjects.

A subsequent analysis was conducted to determine if alternative 4 gene models can also reproduce these three phenotypic features after reclassification. Alternative models #2-8 were used to generate alternative decision trees #2-8. However, none of the alternative models was able to replicate the mortality phenotype, i.e. after reclassification, an independent association between allocation to endotype A and increased odds of mortality was not observed. This in contrast to the result provided by decision tree #1, which was able to replicate the mortality phenotype.

In combination, these results indicate that the presently described simplified decision tree / biomarker-based endotyping strategy is reliable, particularly when model #1 is used. Its relative simplicity makes it amenable for robust and rapid identification of septic shock endotypes and therefore translatable to the critical care environment. This presents a tremendous advantage over the previous 100-gene expression signature, which cannot allow for robust and rapid identification of septic shock endotypes and is not applicable to the critical care environment.

As different subclasses of septic shock have been identified to have different biological activity, with respect to adaptive immunity and glucocorticoid receptor signaling, and differential response to corticosteroid administration, patients classified into the subgroup of patients with increased risk of adverse outcome when prescribed corticosteroids, i.e. endotype A patients as defined herein, can have improved outcomes when treated with non-corticosteroid therapies. Such non-corticosteroid therapies can include alternative therapies and/or high risk therapies. In particular, endotype A patients can be treated with immune enhancing therapies, such as, for example, GMCSF, interleukin-7, anti-PD-1, and the like.

The four genes in decision tree #1, derived from model #1, consist of two protein tyrosine kinases (*JAK2* and *LYN*)*,* one serine- and threonine-specific protein kinase (*PRKCB*)*,* and a guanine nucleotide exchange factor (*SOS2*)*.* All four genes contribute to the GM-CSF signaling pathway. This is important because GM-CSF therapy is under consideration as an adjunctive therapy to prevent or treat sepsis [16-18]. The present data demonstrate that endotype A and B subjects are likely to have different responses to such an approach. Endotyping can therefore play an important role in predictive enrichment for any study of GM-CSF therapy.

Two of the genes (*JAK2* and *SOS2*) correspond to the glucocorticoid receptor signaling pathway, in keeping with previous observations [2, 5]. The biological significance of this is indicated by an independent association between corticosteroid prescription and poor outcome among endotype A subjects, in whom the glucocorticoid receptor signaling pathway genes are repressed relative to the endotype B subjects. After decades of study, the role of adjunctive corticosteroids in septic shock remains controversial [19, 20]. It remains relatively unclear which patients stand to gain the most benefit from adjunctive corticosteroids [21]. The septic shock endotypes can identify patients with septic shock who are more likely to respond to adjunctive corticosteroids; this can be used to estimate corticosteroid responsiveness and therefore inform clinical trial design and even clinical care. In another recent post hoc analysis, it has been demonstrated that combining mortality risk stratification with endotype assignment can be used to identify a subgroup of patients most likely to benefit from corticosteroids (26).

The four genes also correspond to a gene network involved in cell-to-cell signaling and interaction, cellular development, and cellular growth and proliferation. The network contains highly connected nodes corresponding to the pleotropic protein kinases *AKT, ERK1*/*2,* and *JNK.* All three protein kinases have been linked to sepsis-related biology and inflammation [22, 23]. The network is also subject to regulation by miRNA 126a-5p. In a recent report, Fan and colleagues demonstrated in an experimental model of sepsis that endothelial progenitor cells improved vascular function in a manner partially dependent on miRNA 126-5p [24]. These investigators also reported a similar role for miRNA 126-5p in a lipopolysaccharide model of acute lung injury [25].

There is a group of patients in whom differentiation between endotype A and endotype B remains challenging, classified as terminal node four in decision tree #1 described herein. This can be indicative of a septic shock subgroup belonging to third endotype, endotype C. The secondary analysis is limited by the small number of patients allocated to the putative endotype C.

Accordingly, a simplified strategy for identifying septic shock endotypes has been derived. In particular, decision tree #1, derived from model #1 based on a panel of four biomarkers, namely JAK2, LYN, PRKCB, and SOS2, has been demonstrated to be the most effective of the gene combinations evaluated herein. This simplified strategy is amenable to translation to the bedside of critically ill patients, which heretofore has not been possible given the complexity of the previously identified 100-gene signature.

In conclusion, a biomarker-based endotyping strategy has been derived, tested, and validated. This biomarker-based endotyping strategy can be used to stratify patients, to determine an appropriate therapy, or to monitor the therapeutic efficacy of a treatment being administered to a patient with septic shock. This biomarker-based endotyping strategy can be used as an adjunct to physiological assessments for selecting an appropriate therapeutic intervention or monitoring the efficacy of a therapeutic intervention in children with septic shock, where risk of adverse outcome is minimized, or to serve as a surrogate outcome variable in clinical trials.

### Additional Patient Information

The demographic data, clinical characteristics, and/or results from other tests or indicia of septic shock specific to a pediatric patient with septic shock can affect the patient's outcome risk. Accordingly, such demographic data, clinical characteristics, and/or results from other tests or indicia of septic shock can be incorporated into the methods described herein which allow for stratification of individual pediatric patients in order to determine the patient's outcome risk. Such demographic data, clinical characteristics, and/or results from other tests or indicia of septic shock can also be used in combination with the methods described herein which allow for stratification of individual pediatric patients in order to determine the patient's outcome risk.

Such pediatric patient demographic data can include, for example, the patient's age, race, gender, and the like. In some embodiments, the biomarker-based endotyping strategy described herein can incorporate or be used in combination with the patient's age, race, and/or gender to determine an outcome risk.

Such patient clinical characteristics and/or results from other tests or indicia of septic shock can include, for example, the patient's co-morbidities and/or septic shock causative organism, and the like.

Patient co-morbidities can include, for example, acute lymphocytic leukemia, acute myeloid leukemia, aplastic anemia, atrial and ventricular septal defects, bone marrow transplantation, caustic ingestion, chronic granulomatous disease, chronic hepatic failure, chronic lung disease, chronic lymphopenia, chronic obstructive pulmonary disease (COPD), congestive heart failure (NYHA Class IV CHF), Cri du Chat syndrome, cyclic neutropenia, developmental delay, diabetes, DiGeorge syndrome, Down syndrome, drowning, end stage renal disease, glycogen storage disease type 1, hematologic or metastatic solid organ malignancy, hemophagocytic lymphohistiocytosis, hepatoblastoma, heterotaxy, hydrocephalus, hypoplastic left heart syndrome, IPEX Syndrome, kidney transplant, Langerhans cell histiocytosis, liver and bowel transplant, liver failure, liver transplant, medulloblastoma, metaleukodystrophy, mitochondrial disorder, multiple congenital anomalies, multi-visceral transplant, nephrotic syndrome, neuroblastoma, neuromuscular disorder, obstructed pulmonary veins, Pallister Killian syndrome, Prader-Willi syndrome, requirement for chronic dialysis, requirement for chronic steroids, retinoblastoma, rhabdomyosarcoma, rhabdosarcoma, sarcoma, seizure disorder, severe combined immune deficiency, short gut syndrome, sickle cell disease, sleep apnea, small bowel transplant, subglottic stenosis, tracheal stenosis, traumatic brain injury, trisomy 18, type 1 diabetes mellitus, unspecified brain tumor, unspecified congenital heart disease, unspecified leukemia, VATER Syndrome, Wilms tumor, and the like. Any one or more of the above patient co-morbidities can be indicative of the presence or absence of chronic disease in the patient.

Septic shock causative organisms can include, for example, *Acinetobacter baumannii,* Adenovirus, *Bacteroides* species, *Candida* species, *Capnotyophaga jenuni, Cytomegalovirus, Enterobacter cloacae, Enterococcus faecalis, Escherichia coli,* Herpes simplex virus, Human metapneumovirus, Influenza A, *Klebsiella pneumonia, Micrococcus* species, mixed bacterial infection, *Moraxella catarrhalis, Neisseria meningitides,* Parainfluenza, *Pseudomonas* species, *Serratia marcescens, Staphylococcus aureus, Streptococcus agalactiae, Streptococcus milleri, Streptococcus pneumonia, Streptococcus pyogenes,* unspecified gram negative rods, unspecified gram positive cocci, and the like.

In some embodiments, the biomarker-based endotyping strategy described herein can incorporate the patient's co-morbidities to determine an outcome risk. In some embodiments, the biomarker-based endotyping strategy described herein can incorporate the patient's septic shock causative organism to determine an outcome risk.

In some embodiments, the biomarker-based endotyping strategy described herein can be used in combination with the patient's co-morbidities to determine an outcome risk. In some embodiments, the biomarker-based endotyping strategy described herein can be used in combination with the patient's septic shock causative organism to determine an outcome risk.

### Population-Based Risk Scores

A number of models that generate mortality prediction scores based on physiological variables have been developed to date. These can include the PRISM, Pediatric Index of Mortality (PIM), and/ pediatric logistic organ dysfunction (PELOD) models, and the like.

Such models can be very effective for estimating population-based outcome risks but are not intended for stratification of individual patients. The methods described herein which allow for stratification of individual patients can be used alone or in combination with one or more existing population-based risk scores.

In some embodiments, the biomarker-based endotyping strategy described herein can be used with one or more additional population-based risk scores. In some embodiments, the biomarker-based endotyping strategy described herein can be used in combination with PRISM. In some embodiments, the biomarker-based endotyping strategy described herein can be used in combination with PIM. In some embodiments, the biomarker-based endotyping strategy described herein can be used in combination with PELOD. In some embodiments, the biomarker-based endotyping strategy described herein can be used in combination with a population-based risk score other than PRISM, PIM, and PELOD.

### High Risk Therapies

High risk, invasive therapeutic and support modalities can be used to treat septic shock. The methods described herein which allow for the patient's outcome risk to be determined can help inform clinical decisions regarding the application of high risk therapies to specific pediatric patients, based on the patient's outcome risk.

High risk therapies include, for example, extracorporeal membrane oxygenation/life support, plasmapheresis, pulmonary artery catheterization, high volume continuous hemofiltration, and the like. High risk therapies can also include non-corticosteroid therapies, e.g. alternative therapies and/or high risk therapies. In particular, endotype A patients can be treated with immune enhancing therapies, such as, for example, GMCSF, interleukin-7, anti-PD-1, and the like.

In some embodiments, individualized treatment can be provided to a pediatric patient by selecting a pediatric patient classified as high risk by the methods described herein for one or more high risk therapies. In some embodiments, individualized treatment can be provided to a pediatric patient by excluding a pediatric patient classified as low risk from one or more high risk therapies.

Certain embodiments of the disclosure, not being part of the invention include using quantification data from a gene-expression analysis and/or from a mRNA analysis, from a sample of blood, urine, saliva, broncho-alveolar lavage fluid, or the like. Embodiments of the disclosure not being part of the invention include not only methods of conducting and interpreting such tests but also include reagents, compositions, kits, tests, arrays, apparatuses, processing devices, assays, and the like, for conducting the tests. The compositions and kits of the present disclosure can include one or more components which enable detection of the biomarkers disclosed herein and combinations thereof (e.g. the combination of the four biomarkers JAK2, LYN, PRKCB, and SOS2) and can include, but are not limited to, primers, probes, cDNA, enzymes, covalently attached reporter molecules, and the like.

Diagnostic-testing procedure performance is commonly described by evaluating control groups to obtain four critical test characteristics, namely positive predictive value (PPV), negative predictive value (NPV), sensitivity, and specificity, which provide information regarding the effectiveness of the test. The PPV of a particular diagnostic test represents the proportion of positive tests in subjects with the condition of interest (*i*.*e*. proportion of true positives); for tests with a high PPV, a positive test indicates the presence of the condition in question. The NPV of a particular diagnostic test represents the proportion of negative tests in subjects without the condition of interest (*i*.*e*. proportion of true negatives); for tests with a high NPV, a negative test indicates the absence of the condition. Sensitivity represents the proportion of subjects with the condition of interest who will have a positive test; for tests with high sensitivity, a positive test indicates the presence of the condition in question. Specificity represents the proportion of subjects without the condition of interest who will have a negative test; for tests with high specificity, a negative test indicates the absence of the condition.

The threshold for the disease state can alternatively be defined as a 1-D quantitative score, or diagnostic cutoff, based upon receiver operating characteristic (ROC) analysis. The quantitative score based upon ROC analysis can be used to determine the specificity and/or the sensitivity of a given diagnosis based upon subjecting a patient to a decision tree described herein in order to predict an outcome for a pediatric patient with septic shock.

The correlations disclosed herein, between pediatric patient septic shock biomarker levels and/or mRNA levels and/or gene expression levels, provide a basis for conducting a diagnosis of septic shock, or for conducting a stratification of patients with septic shock, or for enhancing the reliability of a diagnosis of septic shock by combining the results of a quantification of a septic shock biomarker with results from other tests or indicia of septic shock. For example, the results of a quantification of one biomarker could be combined with the results of a quantification of one or more additional biomarker, cytokine, mRNA, or the like. Thus, even in situations in which a given biomarker correlates only moderately or weakly with septic shock, providing only a relatively small PPV, NPV, specificity, and/or sensitivity, the correlation can be one indicium, combinable with one or more others that, in combination, provide an enhanced clarity and certainty of diagnosis. Accordingly, the methods and materials of the invention are expressly contemplated to be used both alone and in combination with other tests and indicia, whether quantitative or qualitative in nature.

All examples in the present disclosure are provided as non-limiting examples.

### EXAMPLES

The following non-limiting examples are provided to further illustrate embodiments of the invention disclosed herein. It should be appreciated by those of skill in the art that the techniques disclosed in the examples that follow represent approaches that have been found to function well in the practice of the invention, and thus can be considered to constitute examples of modes for its practice.

### EXAMPLE 1

### Methods

The methods used in the examples that follow are summarized below:

### Study Subjects.

A secondary analysis of previously published data [5] was conducted. The Institutional Review Boards of each of the 18 participating institutions approved the protocol for collection and use of biological specimens and clinical data. Children ≤ 10 years of age admitted to a pediatric intensive care unit (PICU) and meeting pediatric-specific consensus criteria for septic shock were eligible for enrollment [8]. There were no exclusion criteria, other than the inability to obtain informed consent, which was obtained from parents or legal guardians. The consent allows for secondary analyses.

Blood samples were obtained within 24 hours of meeting criteria for septic shock. Clinical and laboratory data were collected daily while in the PICU. Organ failure data were tracked up to day seven of septic shock using previously published criteria [8]. Mortality was tracked for 28 days after enrollment. Illness severity was estimated using the Pediatric Risk of Mortality (PRISM) score [9]. Complicated course was defined as persistence of two or more organ failures at day seven of septic shock or 28-day mortality [5].

Corticosteroid prescription was at the discretion the clinical team caring for the study subjects. Corticosteroid exposure was treated as a binary variable, as previously described [10]. The medication fields of the clinical database were surveyed to determine if the study subjects received systemic corticosteroids. Subjects receiving any formulation of systemic corticosteroids during the first 7 days of meeting criteria for septic shock were classified as being exposed to corticosteroids. The one exception was subjects who received dexamethasone for less than 48 hours for airway edema. These subjects were classified as not being exposed to corticosteroids. All other subjects were classified as not being exposed to corticosteroids. It was not possible to consistently determine dosages or the clinical indications for corticosteroids in all subjects.

### Multiplex mRNA Quantification.

The 100 endotype-defining genes and the 4 housekeeping genes were previously published, along with the gene expression quantification procedures [5]. Gene expression was measured using whole blood-derived RNA obtained within 24 hours of meeting criteria for septic shock. The NanoString nCounter^{™} (NanoString Technologies, Seattle, WA) and a custom-made code set were used. The technology is based on standard hybridization between the target gene, and target-specific capture and reporter probes [11]. All NanoString-based measurements were conducted at the University of Minnesota Genomics Center Core Facility.

### Pathway Analysis.

The genes contributing to the derived decision tree were analyzed using the Ingenuity Pathway Analysis (IPA) platform (Ingenuity Systems, Redwood City, CA) [12, 13]. IPA is a database generated from peer-reviewed literature that provides a tool for discovery of signaling pathways and gene networks represented among uploaded gene lists.

### CART Procedure and Statistical Analysis.

Salford Predictive Modeler v7.0 (Salford Systems, San Diego, CA) was used for the CART procedure [14, 15] to develop a decision tree to accurately predict assignment to endotype A or endotype B, using the smallest possible subset of genes from among the original 100. The primary outcome variable for the modeling procedures was allocation to endotype A as opposed to endotype B. For the purpose of this analysis, the criterion standard for endotype allocation was based on the original strategy using computer assisted image analysis of the 100 endotype defining genes [5]. The modeling procedure considered all 100 genes as candidate predictor variables and used the class probability method. Terminal nodes having less than 5% of the subjects in the root node and terminal nodes that did not improve classification were pruned. Weighting of cases and costs for misclassification were not used in the modeling procedures. Ten-fold cross validation was used to estimate model performance. The code and data used to generate the model is available from the authors.

Other statistical procedures used SigmaStat Software (Systat Software, Inc., San Jose, CA). Initially, data are described using medians, interquartile ranges, frequencies, and percentages. Comparisons between groups used the Mann-Whitney U-test, Chisquare, or Fisher's Exact tests as appropriate. The performance of the decision tree for distinguishing endotypes was measured by constructing receiver operating characteristics curves and calculating diagnostic test characteristics. The association between subclass allocation, outcome, and corticosteroid prescription was modeled using multivariable logistic regression.

### EXAMPLE 2

### Decision Tree #1

The clinical characteristics and demographics of the 300 subjects in the study cohort have been previously published [5]. There were 120 endotype A subjects (40%) and 180 endotype B subjects. Figure 1 shows decision tree #1, derived from model #1, consisting of four genes: *JAK2, PRKCB, SOS2,* and *LYN.* Table 2 provides the annotations for the four genes in decision tree #1. The gene expression values are provided in arbitrary units of mRNA counts, as generated by the NanoString nCounter platform and normalized to four housekeeping genes. The root node provides the total number of subjects originally allocated to endotypes A and B, and their respective rates. Each daughter node provides the respective decision rule criterion based on a gene expression level, and the number of endotype A and B subjects, with the respective rates. Terminal nodes (TN) TN1, TN2, and TN4 contained subjects having a higher probability of being an endotype A (57.1 to 97.4%), whereas TN3, TN5, and TN6 contained subjects having a higher probability of being an endotype B (77.8 to 100%). The 10-fold cross validation procedure yielded an area under the receiver operating curve (AUROC) of 0.90.

**Table 2. Annotations for the four genes in decision tree #1.**

| **GENE Symbol** | **Description** | **Entrez ID Gene** |
|---|---|---|
| JAK2 | Janus kinase 2 | 3717 |
| LYN | LYN proto-oncogene, Src family tyrosine kinase | 4067 |
| PRKCB | Protein kinase C, beta | 5579 |
| SOS2 | SOS Ras/Rho guanine nucleotide exchange factor 2 | 6655 |

Using the original endotype classification strategy as the reference/criterion standard, the area under the receiver operating characteristic curve of decision tree #1 was 0.97 (95% CI: 0.95 to 0.99) for differentiating between endotypes A and B. Subjects allocated to terminal nodes 1, 2, and 4 had a higher probability (57.1 to 97.4%) of being an endotype A, whereas subjects allocated to terminal nodes 3, 5, and 6 had a lower probability (0.0 to 22.2%) of being an endotype A. Accordingly, for calculating the diagnostic test characteristics of decision tree #1, subjects in terminal nodes 1, 2, and 4 were classified as endotype A, and subjects in terminal nodes 3, 5, and 6 were classified as endotype B. Table 3 shows the diagnostic test characteristics of decision tree #1 for identifying endotype A subjects, using the original endotype classification strategy as the reference standard.

**Table 3. Diagnostic test characteristics of decision tree #1 for identifying endotype A.**

| **Variable** | **Value** | **95% C.I.** |
|---|---|---|
| True positives (n) | 113 | -- |
| False positives (n) | 19 | -- |
| True negatives (n) | 161 | -- |
| False negatives (n) | 7 | -- |
| Sensitivity | 94% | 88 to 97 |
| Specificity | 89% | 84 to 93 |
| Positive predictive value | 86% | 78 to 91 |
| Negative predictive value | 96% | 91 to 98 |
| Positive likelihood ratio | 8.9 | 5.8 to 13.7 |
| Negative likelihood ratio | 0.07 | 0.03 to 0.13 |

After deriving decision tree #1 using data from the previously enrolled 300 subjects, as described above, decision tree #1 was tested in 43 newly enrolled subjects. Using the original endotyping strategy, there were 14 endotype A subjects and 29 endotype B subjects in the test cohort. When these subjects were classified according to the derived four-gene decision tree #1, the AUROC was 0.97 (95% CI, 0.93-1.00). The sensitivity and specificity for identifying endotype A were 100% (95% CI, 73-100%) and 79% (95% CI, 60-91%), respectively.

### EXAMPLE 3

### Clinical Characteristics of the Endotypes After Reclassification

Although decision tree #1 correctly classified 91% of the subjects of the original 300 subjects, 19 subjects allocated to endotype A were originally endotype B, and seven subjects allocated to endotype B were originally endotype A. In previous studies using the 100-gene mosaics, endotype A subjects had worse outcomes compared to endotype B subjects, and prescription of corticosteroids was associated with increased risk of mortality among endotype A subjects [5]. Accordingly, it was determined if the classification based on decision tree #1 changed the previously observed differences between endotypes A and B, by combining the derivation and test cohorts (n = 343), and the clinical characteristics of the endotype A and B subjects, as defined by decision tree #1, were compared. Consistent with the previous reports, endotype A subjects were younger, had a higher mortality rate, and had a higher rate of complicated course, compared to endotype B subjects, as shown in Table 4.

**Table 4. Clinical and demographic data for endotypes A and B, as classified by decision tree #1.**

| **Variable** | **Endotype A** | **Endotype B** | **P value** |
|---|---|---|---|
| N | 152 | 191 | -- |
| # of males (%) | 77 (58%) | 96 (57%) | 0.929 |
| Median age, years (IQR) | 1.6 (0.6 - 4.7) | 3.2 (1.4 - 6.6) | <0.001 |
| Median PRISM score (IQR) | 13 (8 - 20) | 11 (8 - 17) | 0.163 |
| Mortality, n (%) | 27 (18) | 15 (8) | 0.009 |
| Complicated course* n (%) | 60 (39) | 41 (21) | <0.001 |

| | | | |
|---|---|---|---|
| Definition of abbreviations: IQR = interquartile range; PRISM = Pediatric Risk of Mortality. *Defined as persistence of two or more organ failures on Day 7 of septic shock or 28-day mortality. | | | |

Table 5 shows the results of logistic regression models evaluating the association between endotype allocation and outcome. After accounting for illness severity (Pediatric Risk of Mortality score) and age, allocation to endotype A was independently associated with increased odds of mortality and complicated course, which represents a composite variable capturing both mortality and organ failure burden.

**Table 5. Logistic regression to test for an association between allocation to endotype A and outcome.**

| **Outcome** | **Variable** | **O.R. (95% C.I.)** | **P value** |
|---|---|---|---|
| Mortality | Allocation to endotype A | 2.3 (1.1 - 4.9) | 0.022 |
| | PRISM score | 1.1 (1.1 - 1.1) | <0.001 |
| | Age | 1.0 (0.9 - 1.2) | 0.562 |
| Complicated course | Allocation to endotype A | 2.1 (1.3 - 3.6) | 0.004 |
| | PRISM score | 1.1 (1.1 - 1.1) | <0.001 |
| | Age | 1.0 (0.9 - 1.1) | 0.514 |

Table 6 shows that prescription of corticosteroids was independently associated with increased odds of mortality among endotype A subjects, but not among endotype B subjects.

**Table 6. Logistic regression to test for an association between corticosteroid prescription and mortality, within endotype.**

| **Endotype** | **Variable** | **O.R. (95% CI)** | **P value** |
|---|---|---|---|
| A | Corticosteroids | 3.7 (1.4 - 9.8) | 0.008 |
| | PRISM score | 1.1 (1.0 - 1.2) | <0.001 |
| | Age | 1.1 (0.9 - 1.2) | 0.449 |
| B | Corticosteroids | 1.3 (0.4 - 4.9) | 0.656 |
| | PRISM score | 1.1 (1.1 - 1.2) | <0.001 |
| | Age | 1.0 (0.9 - 1.2) | 0.743 |

### EXAMPLE 4

### Secondary Considerations

To determine the biological links among the four endotype-defining genes of decision tree #1, these genes were uploaded to the IPA platform. The top scoring canonical pathway represented by these four genes was *granulocyte macrophage colony-stimulating factor signaling* (p = 7. 1E-11). The *glucocorticoid receptor signaling pathway* was also represented among these four genes (p = 0.001).

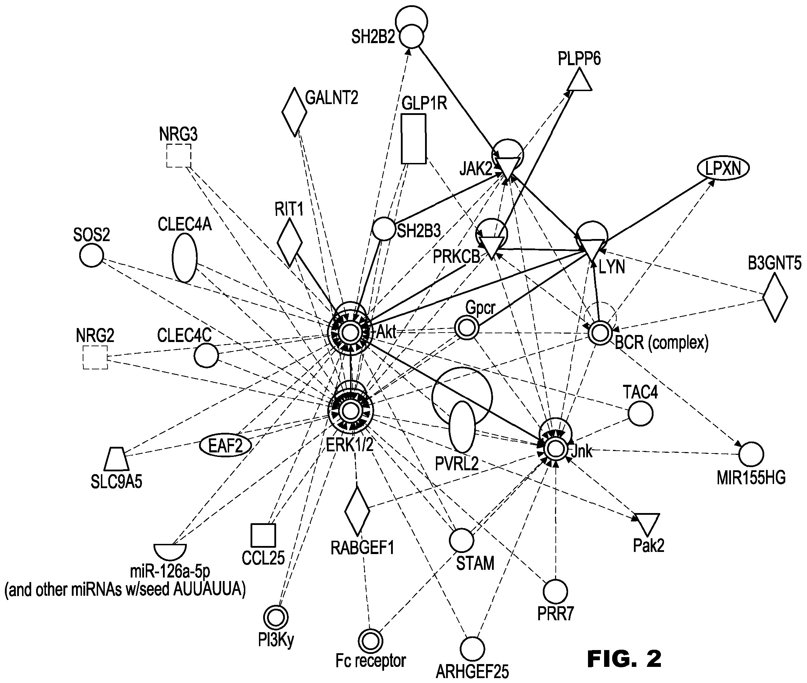

Figure 2 provides the top gene network corresponding to the four endotype-defining genes of decision tree #1. Gray shading highlights the network nodes corresponding to the four classifier genes, *JAK1, PRKCB, SOS2,* and *LYN.* Table 7 provides the annotations for the nodes in the gene network, which contains the protein kinases *AKT, ERK1*/*2,* and *JNK* as highly connected nodes. The network corresponds to cell-to cell signaling and interaction, cellular development, and cellular growth and proliferation, and is subject to regulation by miRNA 126a-5p.

**Table 7. Annotations for the remaining network nodes.**

| **Node ID** | **Description** | **Entrez Gene ID for Human** |
|---|---|---|
| Akt | AKT1/2/3, B/Akt, Pkb, RAC-PK | |
| ARHGEF25 | Rho guanine nucleotide exchange factor 25 | 115557 |
| B3GNT5 | UDP-GIcNAc:betaGal beta-1,3-N-acetylglucosaminyltransferase 5 | 84002 |
| BCR (complex) | B Cell Receptor, Bcr (B-cell receptor complex) | |
| CCL25 | chemokine (C-C motif) ligand 25 | 6370 |
| CLEC4A | C-type lectin domain family 4 member A | 50856 |
| CLEC4C | C-type lectin domain family 4 member C | 170482 |
| EAF2 | ELL associated factor 2 | 55840 |
| ERK1/2 | p44/p42 MAPK | |
| Fc receptor | FCR | |
| GALNT2 | polypeptide N-acetylgalactosaminyltransferase 2 | 2590 |
| GLP1R | glucagon like peptide 1 receptor | 2740 |
| Gper | G-protein-coupled receptor | |
| JAK2 | Janus kinase 2 | 3717 |
| Jnk | JUN Kinase | |
| LPXN | leupaxin | 9404 |
| LYN | LYN proto-oncogene, Src family tyrosine kinase | 4067 |
| miR-126a-5p (and other miRNAs w/seed AUUAUUA) | miR-126-5p | |
| MIR155HG | MIR155 host gene | 114614 |
| NRG2 | neuregulin 2 | 9542 |
| NRG3 | neuregulin 3 | 10718 |
| Pak2 | p21 protein (Cdc42/Rac)-activated kinase 2 | |
| PI3Ky | P13K gamma | |
| PLPP6 | phospholipid phosphatase 6 | 403313 |
| PRKCB | protein kinase C, beta | 5579 |
| PRR7 | proline rich 7 (synaptic) | 80758 |
| PVRL2 | poliovirus receptor-related 2 (herpesvirus entry mediator B) | 5819 |
| RABGEF1 | RAB guanine nucleotide exchange factor (GEF) 1 | 27342 |
| RIT1 | Ras-like without CAAX 1 | 6016 |
| SH2B2 | SH2B adaptor protein 2 | 10603 |
| SH2B3 | SH2B adaptor protein 3 | 10019 |
| SLC9A5 | solute carrier family 9, subfamily A (NHES, cation proton antiporter | 6553 |
| SOS2 | SOS Ras/Rho guanine nucleotide exchange factor 2 | 6655 |
| STAM | signal transducing adaptor molecule | 8027 |
| TAC4 | tachykinin 4 (hemokinin) | 255061 |

Although terminal node 4 of decision tree #1 assigned subjects to endotype A, 43% of these subjects were originally classified as endotype B. Accordingly, some subjects allocated to terminal node 4 of decision tree #1 appear not to be easily defined as endotype A or B. These 21 subjects were grouped as "endotype C", and their clinical phenotype was compared with the remaining endotype A and B subjects. Table 8 shows the results. Endotype A subjects continued to have a higher mortality rate and a higher rate of complicated course, compared to endotype B subjects. Endotype C subjects tended to have an intermediate mortality rate and an intermediate rate of complicated course, relative to both endotypes A and B. Endotype C can therefore be considered an intermediate between the extremes of endotype A and B, or can be considered as a third endotype.

**Table 8. Clinical and demographic data for the endotypes A, B, and C.**

| | **Endotype A** | **Endotype B** | **Endotype C** | **P value** |
|---|---|---|---|---|
| N | 111 | 168 | 21 | -- |
| # of males (%) | 69 (62) | 96 (57) | 8 (38) | 0.120 |
| Median age, years (IQR) | 1.4 (0.3 - 4.2) | 3.0 (1.3 - 6.6) | 3.0 (0.9 - 4.7) | <0.001 |
| Median PRISM score (IQR) | 14 (9 - 21) | 12 (8 - 18) | 15 (9 - 22) | 0.328 |
| Mortality, n (%) | 22 (20) | 13 (8) | 3 (14) | 0.012 |
| Complicated course, n (%) | 47 (42) | 34 (20) | 7 (33) | <0.001 |

### EXAMPLE 5

### Secondary Analysis of Four-Gene Decision Tree #1 for Identifying Septic Shock Endotypes

Septic shock endotypes A and B have been determined based on a 100-gene expression signature, as described above. The 100 endotyping genes reflect adaptive immune function and glucocorticoid receptor signaling, both of which are highly relevant to the pathobiology of septic shock. Importantly, the endotypes differ with respect to outcome, organ failure burden, and treatment responsiveness to corticosteroids. They can also differ with respect to other immune modulating therapies. The clinical utility of endotyping is to enable precision medicine among critically ill patients with septic shock.

CART methodology was applied to reduce the endotype defining gene signature to a decision tree consisting of just four genes, namely decision tree #1, thereby providing for a robust and rapid test for clinical application. A secondary analysis has now been conducted to assess the "uniqueness" of the four-gene decision tree #1 and to consider alternative four-gene combinations.

From the 100 endotyping gene list, four-gene biomarker panels were randomly selected, and a decision tree was derived to differentiate between endotypes A (n = 120) and B (n = 180). The same CART methodology and pruning parameters were applied as used to derive the original four-gene decision tree #1. One hundred iterations of this procedure were performed to generate alternative endotyping models.

Table 9 lists the selected four-gene biomarker panels of the one hundred iterations. These four-gene biomarker panels can be used in classifying a pediatric patient with septic shock as high risk, low risk, or moderate risk, wherein the risk classification can be used to determine, and subsequently administer, an appropriate treatment to the patient.

**Table 9. Table of alternative endotyping models based on alternative four-gene biomarker panels, listed in order of decreasing AUROC for the listed model; the original four-gene decision tree #1, based on model #1, is indicated in italics.**

| **MODEL #** | **GENES** | | | |
|---|---|---|---|---|
| *1* | *JAK2* | *LYN* | *PRKCB* | *SOS2* |
| 2 | GNAI3 | PIK3C3 | TLR1 | TYROBP |
| 3 | CD247 | ITGAX | RHOT 1 | TLR1 |
| 4 | ARPC5 | CSNK1A1 | FCGR2C | PPP2R5C |
| 5 | ASAH1 | FCGR2C | TRA | TYROBP |
| 6 | BTK | FAS | MAP3K3 | PPP2R2A |
| 7 | CASP4 | CD247 | EP300 | MAP3K3 |
| 8 | CREB5 | CSNK1A1 | PRKCB | TLR2 |
| 9 | BCL6 | CASP8 | ITGAM | NCOA2 |
| 10 | CSNK1A1 | ITGAV | MAP3K5 | TBK1 |
| 11 | LAT2 | LYN | PPP2R2A | PRKCB |
| 12 | MAPK1 | PRKAR1A | TLE4 | TLR2 |
| 13 | ARPC5 | CAMK2G | CASP4 | ICAM3 |
| 14 | BCL6 | CAMK2D | PIK3 C3 | SOS1 |
| 15 | BCL6 | LAT2 | PIK3CA | RAF1 |
| 16 | CAMK2D | IL1A | PRKCB | TLR8 |
| 17 | CREB5 | MAP3K7 | MKNK1 | PIK3R1 |
| 18 | JAK2 | PRKCB | PSMB7 | PTPRC |
| 19 | MKNK1 | RAF1 | TBK1 | UBE3A |
| 20 | ASAH1 | BCL6 | PIK3C2A | PIK3R1 |
| 21 | ASAH1 | KAT2B | MDH1 | PPP1R12A |
| 22 | CASP2 | CD79A | FAS | MAP4K4 |
| 23 | CASP4 | GK | MAP3K3 | SOS2 |
| 24 | CASP8 | CD3E | EP300 | PPP2R2A |
| 25 | CD3G | EP300 | LAT2 | TGFBR1 |
| 26 | CREB5 | DBT | MAP2K4 | RHOT 1 |
| 27 | CTNNB1 | DAPP 1 | LYN | PDPR |
| 28 | HDAC4 | MAP4K1 | PIK3CA | PLCG1 |
| 29 | ICAM3 | LYN | TAF11 | TLR8 |
| 30 | MAP2K4 | PRKCB | TLR1 | TNFSF10 |
| 31 | ARPC5 | CTNNB1 | MAP2K4 | PIK3CA |
| 32 | ATP2B2 | HDAC4 | PLCG1 | TBK1 |
| 33 | BCL6 | CASP8 | FCGR2C | TLR1 |
| 34 | BTK | CREB5 | RHOT 1 | ZAP70 |
| 35 | CAMK2D | CREB5 | FCGR2A | PDPR |
| 36 | CAMK2G | INPP5D | MAP2K4 | TBK1 |
| 37 | CD3G | CTNNB1 | MAP3K7 | TLR1 |
| 38 | FCGR2A | MAPK1 | PIK3C2A | SMAD4 |
| 39 | FCGR2C | IL1A | PIK3CD | TLR1 |
| 40 | MAP4K4 | NCOA2 | ROCK1 | TLR1 |
| 41 | BMPR2 | CAMK2G | CASP1 | MAP4K4 |
| 42 | BMPR2 | INPP5D | PDPR | SP1 |
| 43 | CAMK2D | CREB5 | NFATC1 | ZDHHC17 |
| 44 | CAMK2G | MKNK1 | NCOA2 | ZAP70 |
| 45 | ITGAV | MAPK1 | PPP2R2A | PTEN |
| 46 | CAMK4 | DBT | KAT2B | MAP4K4 |
| 47 | CASP1 | MAP2K4 | PPP2R2A | PRKAR1A |
| 48 | CASP4 | JAK1 | MAP3K5 | TLR1 |
| 49 | CD3E | MAP4K4 | MAPK1 | ZAP70 |
| 50 | BTK | CAMK2D | CAMK2G | ROCK1 |
| 51 | CAMK2G | PIK3CA | PTPRC | TRA |
| 52 | MAP4K4 | PLCG1 | PTPRC | ZDHHC17 |
| 53 | NCOA2 | POU2F2 | PPP2R5C | SP1 |
| 54 | ATP2B2 | JAK1 | JAK2 | ROCK1 |
| 55 | CAMK2G | CAMK4 | CD79A | PIK3R1 |
| 56 | CAMK4 | CASP8 | ITGAX | MAP2K4 |
| 57 | CASP4 | DAPP 1 | MAP3K5 | ROCK1 |
| 58 | CASP8 | MAP3K3 | MAP4K4 | PAK2 |
| 59 | DAPP 1 | GNAI3 | MAPK 14 | SEMA4F |
| 60 | DAPP 1 | HDAC4 | MAP3K3 | TNFSF10 |
| 61 | ITGAX | MAP3K7 | MAP4K4 | MAPK1 |
| 62 | JAK2 | PTPRC | SOS2 | ZAP70 |
| 63 | CAMK2G | IL1A | MAP3K3 | TAF11 |
| 64 | CD247 | EP300 | PIAS 1 | TBK1 |
| 65 | DBT | PSMB7 | TNFSF10 | TRA |
| 66 | MKNK1 | POU2F2 | UBE3A | ZAP70 |
| 67 | ARPC5 | NCOA2 | SOS1 | UBE3A |
| 68 | CAMK2G | KAT2B | MAP3K7 | NFATC1 |
| 69 | CD247 | CD79A | FAS | PSMB7 |
| 70 | FAS | JAK1 | MAP4K4 | TLE4 |
| 71 | FCGR2C | ITGAX | PIK3R1 | SEMA4F |
| 72 | HDAC4 | MDH1 | PIK3R1 | PLCG1 |
| 73 | HLA-DMA | INPP5D | MAP2K4 | USP48 |
| 74 | MAP4K4 | PIK3R1 | PPP1R12A | TBK1 |
| 75 | PPP1R12A | PRKCB | TAF11 | TRA |
| 76 | CD247 | MAP3K7 | SEMA4F | TNFSF10 |
| 77 | PLCG1 | PTPRC | SEMA6B | SMAD4 |
| 78 | BMPR2 | CAMK2G | IL1A | MDH1 |
| 79 | BTK | CTNNB1 | GNAI3 | MAP4K4 |
| 80 | CD3E | FAS | MAP3K7 | PRKCB |
| 81 | DAPP 1 | PIK3CA | TAF11 | TNFSF10 |
| 82 | HDAC4 | MAP4K1 | MAPK 14 | SEMA4F |
| 83 | MAP3K 1 | MDH1 | PIAS 1 | USP48 |
| 84 | APAF1 | CD247 | PPP2R2A | TAF11 |
| 85 | APAF1 | EP300 | IL1A | TAF11 |
| 86 | DAPP 1 | FYN | MAP3K3 | SEMA4F |
| 87 | GK | LAT2 | SOS1 | UBE3A |
| 88 | JAK1 | NFATC1 | PDPR | PTEN |
| 89 | ARPC5 | CASP2 | JAK1 | TLR2 |
| 90 | CAMK4 | IL1A | MAP4K1 | POU2F2 |
| 91 | CD3G | INPP5D | PPP1R12A | PPP2R5C |
| 92 | CD79A | CREB1 | FCGR2C | PPP1R12A |
| 93 | INPP5D | ITGAM | JAK1 | NCR3 |
| 94 | ITGAV | NFATC1 | PPP1R12A | USP48 |
| 95 | BTK | CD3G | NCOA2 | ZAP70 |
| 96 | CTNNB1 | MAP4K1 | PAK2 | PIK3 C3 |
| 97 | ATP2B2 | CASP4 | FAS | TBK1 |
| 98 | PSMB7 | ROCK1 | SEMA4F | USP48 |
| 99 | ITGAV | MAP3K7 | PIK3C3 | ZDHHC17 |
| 100 | KAT2B | PDPR | SEMA6B | ZDHHC17 |
| 101 | CAMK2D | PIK3R1 | PPP2R5C | PRKCB |

### EXAMPLE 6

### Phenotypes Based on Alternative Endotyping Models

An analysis was conducted to determine if alternative 4 gene models listed in Table 9 can also reproduce these three phenotypic features of endotypes A, B, and C after reclassification. Table 10 below provides the odds ratios for mortality and complicated course among patients allocated to endotype A. It can be assumed that the odds ratios for mortality and complicated course among patients allocated to endotype B are the inverse of that provided in Table 10. Each odds ratio reflects a multivariable logistic regression procedure that takes into account the confounding variables of age, baseline illness severity, and co-morbidity burden. Model #1, indicated in italics, reflects the primary model described in the preceding examples. The remaining models reflect the alternative four-gene models #2-8 from Table 9 previously generated via random selection of four gene sets.

**Table 10.**

| **MODEL #** | **Outcome** | **O.R.** | **95% C.I.** | **P value** |
|---|---|---|---|---|
| #1 | *Mortality* | *2*.*3* | *1.0 to 4.9* | *0.022* |
| | *Complicated Course* | *2.1* | *1.3 to 3.6* | *0.004* |
| #2 | Mortality | 1.9 | 0.9 to 3.9 | 0.101 |
| | Complicated Course | 2 | 1.2 to 3.3 | 0.013 |
| #3 | Mortality | 1.9 | 0.9 to 4.0 | 0.09 |
| | Complicated Course | 2.4 | 1.4 to 4.0 | 0.001 |
| #4 | Mortality | 1.8 | 0.8 to 3.6 | 0.134 |
| | Complicated Course | 1.4 | 0.8 to 2.4 | 0.18 |
| #5 | Mortality | 1.9 | 0.9 to 3.9 | 0.105 |
| | Complicated Course | 2.1 | 1.1 to 3.9 | 0.021 |
| #6 | Mortality | 2.1 | 0.98 to 4.4 | 0.056 |
| | Complicated Course | 2.6 | 1.5 to 4.4 | <0.001 |
| #7 | Mortality | 1.21 | 0.6 to 2.6 | 0.562 |
| | Complicated Course | 2 | 1.2 to 3.5 | 0.007 |
| #8 | Mortality | 1.7 | 0.8 to 3.5 | 0.179 |
| | Complicated Course | 1.8 | 1.1 to 3.0 | 0.032 |

Table 11 below provides the odds ratio for mortality in patients who receive corticosteroids, within each endotype. Each odds ratio reflects a multivariable logistic regression procedure that takes into account the confounding variables of age, baseline illness severity, and co-morbidity burden. Model #1, indicated in italics, reflects the primary model described in the preceding examples. The remaining models reflect alternative 4-gene models #2-8 from Table 9 previously generated via random selection of four-gene sets.

**Table 11.**

| **MODEL #** | **Endotype** | **O.R.** | **95% C.I.** | **P value** |
|---|---|---|---|---|
| #*1* | *A* | *3*.*7* | *1.4 to 9.8* | *0.008* |
| | *B* | *1.8* | *0.5 to 6.1* | *0.366* |
| #2 | A | 5.4 | 1.8 to 16.0 | 0.002 |
| | B | 1.1 | 0.4 to 3.1 | 0.918 |
| #3 | A | 4.1 | 1.6 to 10.6 | 0.004 |
| | B | 1.5 | 0.44 to 5.0 | 0.515 |
| #4 | A | 5.3 | 1.8 to 15.8 | 0.003 |
| | B | 1.4 | 0.5 to 4.0 | 0.567 |
| #5 | A | 3.4 | 1.3 to 9.3 | 0.015 |
| | B | 1.9 | 0.6 to 6.2 | 0.259 |
| #6 | A | 2.1 | 1.4 to 12.3 | 0.008 |
| | B | 2.3 | 0.7 to 6.9 | 0.156 |
| #7 | A | 2.1 | 0.8 to 5.4 | 0.135 |
| | B | 4.1 | 1.0 to 15.9 | 0.044 |
| #8 | A | 3.3 | 1.2 to 9.0 | 0.023 |
| | B | 1.9 | 0.6 to 5.9 | 0.241 |

None of alternative models #2-8 was demonstrated to replicate the mortality phenotype. In other words, after reclassification, an independent association between allocation to endotype A and increased odds of mortality was not observed. This is a fundamental aspect of the previously described results. All of the alternative models listed in Tables 10-11, with the exception of model #4, were shown to replicate the complicated course phenotype. All of the alternative models listed in Tables 10-11, with the exception of model #7, were shown to replicate the corticosteroid phenotype. Thus, while close, none of the alternative four-gene models #2-8 has demonstrated the ability to fully replicate the phenotypes previously reported and confirmed by model #1.

Based on this, further testing of the alternative four-gene models listed in Table 9 was not performed. Such models would be expected to be even less able to fully replicate the previously reported phenotypes.

Model #1 therefore most closely approximates the endotyping procedure based on 100 genes, which is the reference criterion. Model #1 has the best statistical performance during derivation and validation, and also completely replicates the phenotypic features of the endotypes, when compared to the reference criterion.

In a subsequent analysis, the 25 unique genes that appear in models #1 through #8 were used as candidate predictor variables. This is the most objective approach to assessing which of the genes are the "best" for differentiating between endotype A and B. Notably, this procedure replicated model #1, as shown in Figure 4, which depicts an alternative version of decision tree #1.

### EXAMPLE 7

### Methods of Classifying and Treating Patients Based on 4-Gene Signature

The biomarkers described herein are used to classify a patient with septic shock as having high risk or low risk of adverse outcome. First, a sample from a pediatric patient with septic shock is analyzed to determine the expression levels of two or more biomarkers selected from the biomarkers listed in Table 1. For example, all of the biomarkers in model #1, namely JAK2, LYN, PRKCB, and SOS2, are analyzed, and decision tree #1, as described herein, is applied to the resulting biomarker levels. It is then determined whether the expression level(s) of the two or more biomarkers are elevated above a cut-off level, wherein the patient is classified as endotype A/high risk where there is a presence of a non-elevated level of the two or more biomarkers, and wherein the patient is classified as endotype B/low risk or endotype C/intermediate risk where there is an absence of an non-elevated level of the two or more biomarkers.

The patient is then administered a treatment based on the patient's classification as endotype A/high risk, endotype B/low risk, or endotype C/intermediate risk. A patient that is classified as endotype B/low risk or endotype C/intermediate risk is administered a treatment which includes one or more corticosteroid, whereas a patient that is classified as endotype A/high risk is administered a treatment which excludes one or more corticosteroid. A patient that is classified as endotype A/high risk is then optionally administered a high risk treatment or therapy. In this way, individualized treatment is provided, and clinical outcomes for septic shock patients are improved and morbidity reduced.

A second sample is then obtained, and the patient's classification is confirmed or updated. The treatment being administered is then maintained or revised as necessary. This allows for determination and monitoring of therapeutic efficacy.

The various methods and techniques described above provide a number of ways to carry out the application. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

In some embodiments, the numbers expressing quantities of ingredients, properties such as molecular weight, reaction conditions, and so forth, used to describe and claim certain embodiments of the application are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In some embodiments, the terms "a" and "an" and "the" and similar references used in the context of describing a particular embodiment of the application (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. No language in the specification should be construed as indicating any non-claimed element essential to the practice of the application.

Preferred embodiments of this application are described herein, including the best mode known to the inventors for carrying out the application. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

In closing, it is to be understood that the embodiments of the application disclosed herein are illustrative of the principles of the embodiments of the application. Thus, by way of example, but not of limitation, alternative configurations of the embodiments of the application can be utilized in accordance with the teachings herein. Accordingly, embodiments of the present application are not limited to that precisely as shown and described.

### References

1. Cohen J, Vincent JL, Adhikari NK, Machado FR, Angus DC, Calandra T, Jaton K, Giulieri S, Delaloye J, Opal S et al.: Sepsis: a roadmap for future research. The Lancet Infectious Diseases 2015, 15(5):581-614.
2. Wong HR, Cvijanovich N, Lin R, Allen GL, Thomas NJ, Willson DF, Freishtat RJ, Anas N, Meyer K, Checchia PA et al.: Identification of pediatric septic shock subclasses based on genome-wide expression profiling. BMC Medicine 2009, 7:34.
3. Wong HR, Cvijanovich NZ, Allen GL, Thomas NJ, Freishtat RJ, Anas N, Meyer K, Checchia PA, Lin R, Shanley TP et al.: Validation of a gene expression-based subclassification strategy for pediatric septic shock. Critical Care Medicine 2011, 39(11):2511-2517.
4. Wong HR, Wheeler DS, Tegtmeyer K, Poynter SE, Kaplan JM, Chima RS, Stalets E, Basu RK, Doughty LA: Toward a clinically feasible gene expression-based subclassification strategy for septic shock: proof of concept. Critical Care Medicine 2010, 38(10):1955-1961,
5. Wong HR, Cvijanovich NZ, Anas N, Allen GL, Thomas NJ, Bigham MT, Weiss SL, Fitzgerald J, Checchia PA, Meyer K et al.: Developing a clinically feasible personalized medicine approach to pediatric septic shock. Am J Respir Crit Care Med 2015, 191(3):309-315.
6. Wong HR, Atkinson SJ, Cvijanovich NZ, Anas N, Allen GL, Thomas NJ, Bigham MT, Weiss SL, Fitzgerald JC, Checchia PA et al.: Combining prognostic and predictive enrichment strategies to identify children with septic shock responsive to corticosteroids. Critical Care Medicine 2016.
7. Maslove DM, Wong HR: Gene expression profiling in sepsis: timing, tissue, and translational considerations. Trends in Molecular Medicine 2014, 20(4):204-213.
8. Wong HR, Shanley TP, Sakthivel B, Cvijanovich N, Lin R, Allen GL, Thomas NJ, Doctor A, Kalyanaraman M, Tofil NM et al.: Genome-level expression profiles in pediatric septic shock indicate a role for altered zinc homeostasis in poor outcome. Physiological Genomics 2007, 30(2): 146-155.
9. Pollack MM, Patel KM, Ruttimann UE: The Pediatric Risk of Mortality III--Acute Physiology Score (PRISM III-APS): a method of assessing physiologic instability for pediatric intensive care unit patients. The Journal of Pediatrics 1997, 131(4):575-581.
10. Atkinson SJ, Cvijanovich NZ, Thomas NJ, Allen GL, Anas N, Bigham MT, Hall M, Freishtat RJ, Sen A, Meyer K et al.: Corticosteroids and pediatric septic shock outcomes: a risk stratified analysis. PloS One 2014, 9(11):e112702.
11. Geiss GK, Bumgarner RE, Birditt B, Dahl T, Dowidar N, Dunaway DL, Fell HP, Ferree S, George RD, Grogan T et al.: Direct multiplexed measurement of gene expression with color-coded probe pairs. Nature Biotechnology 2008, 26(3):317-325.
12. Wynn JL, Cvijanovich NZ, Allen GL, Thomas NJ, Freishtat RJ, Anas N, Meyer K, Checchia PA, Lin R, Shanley TP et al.: The influence of developmental age on the early transcriptomic response of children with septic shock. Mol Med 2011, 17(11-12):1146-1156.
13. Weiss SL, Cvijanovich NZ, Allen GL, Thomas NJ, Freishtat RJ, Anas N, Meyer K, Checchia PA, Shanley TP, Bigham MT et al.: Differential expression of the nuclear-encoded mitochondrial transcriptome in pediatric septic shock. Crit Care 2014, 18(6):623.
14. Che D, Liu Q, Rasheed K, Tao X: Decision tree and ensemble learning algorithms with their applications in bioinformatics. Advances in Experimental Medicine and Biology 2011, 696:191-199.
15. Muller R, Mockel M: Logistic regression and CART in the analysis of multimarker studies. Clinica Chimica Acta; International Journal of Clinical Chemistry 2008, 394(1-2):1-6.
16. Mathias B, Szpila BE, Moore FA, Efron PA, Moldawer LL: A Review of GM-CSF Therapy in Sepsis. Medicine 2015, 94(50):e2044.
17. Marlow N, Morris T, Brocklehurst P, Carr R, Cowan F, Patel N, Petrou S, Redshaw M, Modi N, Dore CJ: A randomised trial of granulocyte-macrophage colony-stimulating factor for neonatal sepsis: childhood outcomes at 5 years. Archives of Disease in Childhood Fetal and Neonatal Edition 2015, 100(4):F320-326.
18. Hutchins NA, Unsinger J, Hotchkiss RS, Ayala A: The new normal: immunomodulatory agents against sepsis immune suppression. Trends in Molecular Medicine 2014, 20(4):224-233.
19. Sprung CL, Annane D, Keh D, Moreno R, Singer M, Freivogel K, Weiss YG, Benbenishty J, Kalenka A, Forst H et al.: Hydrocortisone therapy for patients with septic shock. The New England Journal of Medicine 2008, 358(2): 111-124.
20. Annane D, Sebille V, Charpentier C, Bollaert PE, Francois B, Korach JM, Capellier G, Cohen Y, Azoulay E, Troche G et al.: Effect of treatment with low doses of hydrocortisone and fludrocortisone on mortality in patients with septic shock. Jama 2002, 288(7):862-871.
21. Atkinson SJ, Wong HR: Identifying critically ill patients who may benefit from adjunctive corticosteroids: not as easy as we thought. Pediatric Critical Care Medicine : A Journal of the Society of Critical Care Medicine and the World Federation of Pediatric Intensive and Critical Care Societies 2014, 15(8):769-771.
22. Manukyan MC, Weil BR, Wang Y, Abarbanell AM, Herrmann JL, Poynter JA, Meldrum DR: The phosphoinositide-3 kinase survival signaling mechanism in sepsis. Shock 2010, 34(5):442-449.
23. Sabio G, Davis RJ: TNF and MAP kinase signalling pathways. Seminars in Immunology 2014, 26(3):237-245.
24. Fan H, Goodwin AJ, Chang E, Zingarelli B, Borg K, Guan S, Halushka PV, Cook JA: Endothelial progenitor cells and a stromal cell-derived factor-lalpha analogue synergistically improve survival in sepsis. Am J Respir Crit Care Med 2014, 189(12):1509-1519.
25. Guo C, Goodwin AJ, Buie JN, Cook JA, Halushka PV, Argraves K, Zingarelli B, Zhang XK, Wang L, Fan H: A Stromal Cell-derived Factor 1 alpha Analogue Improves Endothelial Cell Function in Lipopolysaccharide-induced Acute Respiratory Distress Syndrome. Mol Med 2016, 22:115-123.
26. Wong HR, Atkinson SJ, Cvijanovich NZ, Anas N, Allen GL, Thomas NJ, Bigham MT, Weiss SL, Fitzgerald JC, Checchia PA, et al. Combining prognostic and predictive enrichment strategies to identify children with septic shock responsive to corticosteroids. Crit Care Med 2016; 44:e1000-1003.

## Claims

1. An in vitro method of classifying a pediatric patient with septic shock as high risk or low risk of adverse outcome, the method comprising:
analyzing a sample from a pediatric patient with septic shock to determine the expression levels of two or more biomarkers selected from the group consisting of JAK2, LYN, PRKCB, and SOS2;
determining whether the expression levels of the two or more biomarkers are elevated above a cut-off level; and
classifying the patient as endotype A / high risk or other than endotype A / high risk, wherein a classification of endotype A / high risk comprises:
a) a non-elevated level of JAK2 and a non-elevated level of PRKCB; or
b) a non-elevated level of JAK2, an elevated level of PRKCB, and a non-elevated level of LYN;
and wherein a classification other than endotype A / high risk comprises:
c) a non-elevated level of JAK2, an elevated level of PRKCB, and an elevated level of LYN; or
d) an elevated level of JAK2, a non-elevated level of SOS2, and a highly elevated level of LYN; or
e) elevated levels of JAK2 and SOS2; or
f) an elevated level of JAK2, a non-elevated level of SOS2, and a non-highly elevated level of LYN.

2. The method of claim 1, wherein biomarker expression levels are determined by mRNA quantification, optionally wherein biomarker expression levels are determined by normalized mRNA counts and/or by cycle threshold (CT) values.

3. The method of claim 1, wherein the biomarkers comprise a pair of biomarkers selected from the group consisting of: JAK2 and LYN; JAK2 and PRKCB; JAK2 and SOS2; LYN and PRKCB; LYN and SOS2; and PRKCB and SOS2; or
wherein the biomarkers comprise three or more selected from the group consisting of JAK2, LYN, PRKCB, and SOS2; or
wherein the biomarkers comprise a trio of biomarkers selected from the group consisting of: JAK2, LYN, and PRKCB; JAK2, LYN, and SOS2; JAK2, PRKCB, and SOS2; LYN, PRKCB, and SOS2; or
wherein the biomarkers comprise all of JAK2, LYN, PRKCB, and SOS2.

4. The method of any of claims 1 to 3, wherein biomarker levels are determined by normalized mRNA counts, and wherein:
a) an elevated level of JAK2 corresponds to a serum JAK2 mRNA count greater than 1780;
b) an elevated level of PRKCB corresponds to a serum PRKCB mRNA count greater than 990;
c) an elevated level of SOS2 corresponds to a serum SOS2 mRNA count greater than 480;
d) an elevated level of LYN corresponds to a serum LYN mRNA count greater than 870; and
e) a highly elevated level of LYN corresponds to a serum LYN mRNA count greater than 940;
optionally comprising application of the decision tree of Figure 1.

5. The method of any of claims 1 to 3, wherein biomarker levels are determined by normalized mRNA counts, and wherein:
a) an elevated level of JAK2 corresponds to a serum JAK2 mRNA count greater than 1784;
b) an elevated level of PRKCB corresponds to a serum PRKCB mRNA count greater than 986;
c) an elevated level of SOS2 corresponds to a serum SOS2 mRNA count greater than 480;
d) an elevated level of LYN corresponds to a serum LYN mRNA count greater than 873; and
e) a highly elevated level of LYN corresponds to a serum LYN mRNA count greater than 938;
optionally comprising application of the decision tree of Figure 4.

6. The method of any one of claims 1-5, wherein the determination of whether the levels of the two or more biomarkers are non-elevated or elevated above a cut-off level comprises applying the biomarker expression level data to a decision tree comprising the two or more biomarkers.

7. The method of any one of claims 1-6, wherein a classification other than endotype A / high risk comprises a classification of endotype B / low risk or endotype C / moderate risk.

8. The method of any one of claims 1-7, wherein the determination of whether the levels of the two or more biomarkers are non-elevated or elevated above a cutoff level is combined with one or more patient demographic data and/or clinical characteristics and/or results from other tests or indicia of septic shock; optionally
wherein the patient demographic data and/or clinical characteristics and/or results from other tests or indicia of septic shock comprise at least one selected from the group consisting of the septic shock causative organism, the presence or absence or chronic disease, and/or the age, gender, race, and/or co-morbidities of the patient.

9. The method of any one of claims 1-8, wherein the determination of whether the levels of the two or more biomarkers are non-elevated or elevated above a cut-off level is combined with one or more additional population-based risk scores; optionally wherein the one or more population-based risk scores comprises at least one selected from the group consisting of Pediatric Risk of Mortality (PRISM), Pediatric Index of Mortality (PIM), and/or Pediatric Logistic Organ Dysfunction (PELOD).

10. The method of any one of claims 1-9, wherein the sample has been obtained within the first hour of presentation with septic shock; or
wherein the sample has been obtained within the first 48 hours of presentation with septic shock.

11. The method of any one of claims 1-10, further comprising identifying a patient that is not endotype A / high risk and is a candidate for a treatment comprising one or more corticosteroid, or identifying a patient that is classified as endotype A / high risk and is a candidate for a treatment comprising one or more therapy excluding a corticosteroid; optionally
wherein the treatment for the patient that is classified as endotype A / high risk comprises one or more high risk therapy; optionally
wherein the treatment for the patient that is endotype A / high risk comprises one or more high risk therapy selected from the group consisting of immune enhancing therapy, extracorporeal membrane oxygenation/life support, plasmapheresis, pulmonary artery catheterization, and/or high volume continuous hemofiltration; optionally
wherein the immune enhancing therapy comprises GMCSF, interleukin-7, and/or anti-PD-1.

12. The method of claim 11, wherein a treatment comprising one or more corticosteroid is provided to a patient that is not endotype A / high risk, or a treatment comprising one or more therapy excluding a corticosteroid is provided to a patient that is classified as endotype A / high risk, thereby improving an outcome in a pediatric patient with septic shock.

13. The method of claim 11 or 12, further comprising:
in a sample obtained from the patient at a later time point, analyzing the subsequent sample to determine the expression levels of the two or more biomarkers selected from the group consisting of JAK2, LYN, PRKCB, and SOS2, and determining whether the expression levels of the two or more biomarkers are non-elevated or elevated above a cut-off level, thereby determining a change to the patient's endotype classification; and
determining that treatment should be maintained if the patient's endotype classification has not changed, or that the treatment should be changed if the patient's endotype classification has changed.

14. Use of a diagnostic kit, test, or array comprising a reporter hybridization probe, and a capture hybridization probe specific for each of two or more mRNA markers selected from the group consisting of JAK2, LYN, PRKCB, and SOS2, in a method according to claim 1; optionally
wherein the mRNA markers comprise JAK2, LYN, PRKCB, and SOS2.

15. The use according to claim 14, wherein the diagnostic kit, test, or array, further comprises a collection cartridge for immobilization of the hybridization probes; optionally
wherein the reporter and the capture hybridization probes comprise signal and barcode elements, respectively.

## Patentansprüche

1. In-vitro-Verfahren zum Klassifizieren eines pädiatrischen Patienten mit septischem Schock als hohes Risiko oder geringes Risiko eines ungünstigen Ausgangs, das Verfahren umfassend:
Analysieren einer Probe von einem pädiatrischen Patienten mit septischem Schock, um die Expressionsspiegel von zwei oder mehr Biomarkern zu bestimmen, die aus der Gruppe ausgewählt sind, bestehend aus JAK2, LYN, PRKCB und SOS2;
Bestimmen, ob die Expressionsspiegel der zwei oder mehr Biomarker über einen Grenzspiegel erhöht sind; und
Klassifizieren des Patienten als Endotyp A / hohes Risiko oder anders als Endotyp A / hohes Risiko, wobei eine Klassifikation von Endotyp A / hohes Risiko umfasst:
a) einen nicht erhöhten Spiegel von JAK2 und einen nicht erhöhten Spiegel von PRKCB; oder
b) einen nicht erhöhten Spiegel von JAK2, einen erhöhten Spiegel von PRKCB und einen nicht erhöhten Spiegel von LYN;
und wobei eine Klassifikation anders als Endotyp A / hohes Risiko umfasst:
c) einen nicht erhöhten Spiegel von JAK2, einen erhöhten Spiegel von PRKCB und einen erhöhten Spiegel von LYN; oder
d) einen erhöhten Spiegel von JAK2, einen nicht erhöhten Spiegel von SOS2 und einen stark erhöhten Spiegel von LYN; oder
e) einen erhöhten Spiegel von JAK2 und SOS2; oder
f) einen erhöhten Spiegel von JAK2, einen nicht erhöhten Spiegel von SOS2 und einen nicht stark erhöhten Spiegel von LYN.

2. Verfahren nach Anspruch 1, wobei Biomarkerexpressionsspiegel durch mRNA-Quantifizierung bestimmt werden, optional wobei Biomarkerexpressionsspiegel durch normalisierte mRNA-Zählungen und/oder durch Zyklusschwellenwerte (CT-Werte) bestimmt werden.

3. Verfahren nach Anspruch 1, wobei die Biomarker ein Paar Biomarker umfassen, die aus der Gruppe ausgewählt sind, bestehend aus: JAK2 und LYN; JAK2 und PRKCB; JAK2 und SOS2; LYN und PRKCB; LYN und SOS2; und PRKCB und SOS2; oder
wobei die Biomarker drei oder mehr umfassen, die aus der Gruppe ausgewählt sind, bestehend aus JAK2, LYN, PRKCB und SOS2; oder
wobei die Biomarker ein Trio von Biomarkern umfassen, das aus der Gruppe ausgewählt ist, bestehend aus: JAK2, LYN und PRKCB; JAK2, LYN und SOS2; JAK2, PRKCB und SOS2; LYN, PRKCB und SOS2; oder
wobei die Biomarker alle von JAK2, LYN, PRKCB und SOS2 umfassen.

4. Verfahren nach einem der Ansprüche 1 bis 3, wobei Biomarkerspiegel durch normalisierte mRNA-Zählungen bestimmt werden, und wobei:
a) ein erhöhter Spiegel von JAK2 einer Serum-JAK2-mRNA-Zählung von über 1780 entspricht;
b) ein erhöhter Spiegel von PRKCB einer Serum-PRKCB-mRNA-Zählung von über 990 entspricht;
c) ein erhöhter Spiegel von SOS2 einer Serum-SOS2-mRNA-Zählung von über 480 entspricht;
d) ein erhöhter Spiegel von LYN einer Serum-LYN-mRNA-Zählung von über 870 entspricht; und
e) ein stark erhöhter Spiegel von LYN einer Serum-LYN-mRNA-Zählung von über 940 entspricht;
optional umfassend eine Anwendung des Entscheidungsbaums von Figur 1.

5. Verfahren nach einem der Ansprüche 1 bis 3, wobei Biomarkerspiegel durch normalisierte mRNA-Zählungen bestimmt werden, und wobei:
a) ein erhöhter Spiegel von JAK2 einer Serum-JAK2-mRNA-Zählung von über 1784 entspricht;
b) ein erhöhter Spiegel von PRKCB einer Serum-PRKCB-mRNA-Zählung von über 986 entspricht;
c) ein erhöhter Spiegel von SOS2 einer Serum-SOS2-mRNA-Zählung von über 480 entspricht;
d) ein erhöhter Spiegel von LYN einer Serum-LYN-mRNA-Zählung von über 873 entspricht; und
e) ein stark erhöhter Spiegel von LYN einer Serum-LYN-mRNA-Zählung von über 938 entspricht;
optional umfassend die Anwendung des Entscheidungsbaums von Figur 4.

6. Verfahren nach einem der Ansprüche 1 bis 5, wobei die Bestimmung, ob die Spiegel der zwei oder mehr Biomarker über einen Grenzspiegel nicht erhöht oder erhöht sind, ein Anwenden der Biomarkerexpressionsspiegeldaten auf einen Entscheidungsbaum umfasst, umfassend die zwei oder mehr Biomarker.

7. Verfahren nach einem der Ansprüche 1 bis 6, wobei eine Klassifikation anders als Endotyp A / hohes Risiko eine Klassifikation von Endotyp B / geringes Risiko oder Endotyp C / moderates Risiko umfasst.

8. Verfahren nach einem der Ansprüche 1 bis 7, wobei die Bestimmung, ob die Spiegel der zwei oder mehr Biomarker über einen Grenzspiegel nicht erhöht oder erhöht sind, mit einem oder mehreren Patientendemografiedaten und/oder klinischen Merkmalen und/oder Ergebnissen von anderen Tests oder Zeichen des septischen Schocks kombiniert wird; optional
wobei die Patientendemografiedaten und/oder die klinischen Merkmale und/oder die Ergebnisse von anderen Tests oder die Zeichen des septischen Schocks mindestens eines umfassen, das aus der Gruppe ausgewählt ist, bestehend aus dem Organismus, der den septischen Schock verursacht, dem Vorhandensein oder Nichtvorhandensein einer chronischen Erkrankung und/oder dem Alter, dem Geschlecht, der Ethnie und/oder den Komorbiditäten des Patienten.

9. Verfahren nach einem der Ansprüche 1 bis 8, wobei die Bestimmung, ob die Spiegel der zwei oder mehr Biomarker über einen Grenzspiegel nicht erhöht oder erhöht sind, mit einem oder mehreren zusätzlichen bevölkerungsbasierten Risikowerten kombiniert wird; optional wobei der eine oder die mehreren bevölkerungsbasierten Risikowerte mindestens eines umfassen, das aus der Gruppe ausgewählt ist, bestehend aus einem Kindersterblichkeitsrisiko (PRISM), einem Kindersterblichkeitsindex (PIM) und/oder einer pädiatrischen logistischen Organdysfunktion (PELOD).

10. Verfahren nach einem der Ansprüche 1 bis 9, wobei die Probe innerhalb der ersten Stunde eines Auftretens von septischem Schock erhalten wurde; oder
wobei die Probe innerhalb der ersten 48 Stunden des Auftretens von septischem Schock erhalten wurde.

11. Verfahren nach einem der Ansprüche 1 bis 10, ferner umfassend ein Identifizieren eines Patienten, der nicht Endotyp A / hohes Risiko ist und ein Kandidat für eine Behandlung ist, umfassend ein oder mehrere Corticosteroide, oder das Identifizieren eines Patienten, der als Endotyp A / hohes Risiko klassifiziert ist und ein Kandidat für eine Behandlung ist, umfassend eine oder mehrere Therapien ohne ein Corticosteroid; optional
wobei die Behandlung für den Patienten, der als Endotyp A / hohes Risiko klassifiziert ist, eine oder mehrere Therapien mit hohem Risiko umfasst; optional
wobei die Behandlung für den Patienten, der Endotyp A / hohes Risiko ist, eine oder mehrere Therapien mit hohem Risiko umfasst, die aus der Gruppe ausgewählt sind, bestehend aus einer immunverstärkenden Therapie, einer extrakorporalen Membranoxygenierung/Lebenserhaltung, Plasmapherese, einer Katheterisierung einer Lungenarterie und/oder einer kontinuierlichen Hämofiltration mit hohem Volumen; optional
wobei die immunverstärkende Therapie GMCSF, Interleukin-7 und/oder Anti-PD-1 umfasst.

12. Verfahren nach Anspruch 11, wobei eine Behandlung, umfassend ein oder mehrere Corticosteroide, an einen Patienten bereitgestellt wird, der nicht Endotyp A / hohes Risiko ist, oder eine Behandlung, umfassend eine oder mehrere Therapien ohne ein Corticosteroid, an einen Patienten bereitgestellt wird, der als Endotyp A / hohes Risiko klassifiziert ist, wobei dadurch ein Ausgang bei einem pädiatrischen Patienten mit septischem Schock verbessert wird.

13. Verfahren nach Anspruch 11 oder 12, ferner umfassend:
in einer Probe, die von dem Patienten zu einem späteren Zeitpunkt erhalten wird, Analysieren der nachfolgenden Probe, um die Expressionsspiegel der zwei oder mehr Biomarker zu bestimmen, die aus der Gruppe ausgewählt sind, bestehend aus JAK2, LYN, PRKCB und SOS2, und Bestimmen, ob die Expressionsspiegel der zwei oder mehr Biomarker über einen Grenzspiegel nicht erhöht oder erhöht sind, wobei dadurch eine Änderung einer Endotypklassifikation des Patienten bestimmt wird; und
Bestimmen, dass die Behandlung aufrechterhalten werden sollte, falls die Endotypklassifikation des Patienten sich nicht geändert hat, oder dass die Behandlung geändert werden sollte, falls sich die Endotypklassifikation des Patienten geändert hat.

14. Verwendung eines/einer diagnostischen Kits, Tests oder Anordnung, umfassend eine Sonde für Reporterhybridisierung und eine Sonde für Einfanghybridisierung, die speziell für jeden von zwei oder mehr mRNA-Markern ist, die aus der Gruppe ausgewählt sind, bestehend aus JAK2, LYN, PRKCB und SOS2, in einem Verfahren gemäß Anspruch 1; optional
wobei die mRNA-Marker JAK2, LYN, PRKCB und SOS2 umfassen.

15. Verwendung nach Anspruch 14, wobei das/der/die diagnostische Kit, Test oder Anordnung ferner eine Sammelkartusche für eine Immobilisierung der Hybridisierungssonden umfasst; optional
wobei die Sonde für Reporterhybridisierung und die Sonde für Einfanghybridisierung jeweils Signal- und Barcode-Elemente umfassen.

## Revendications

1. Procédé in vitro de classification d'un patient pédiatrique en choc septique comme étant à risque élevé ou à risque faible d'issue défavorable, le procédé comprenant :
l'analyse d'un échantillon provenant d'un patient pédiatrique en choc septique pour déterminer les niveaux d'expression de deux biomarqueurs ou plus choisis dans le groupe constitué par JAK2, LYN, PRKCB et SOS2 ;
le fait de déterminer si les niveaux d'expression des deux biomarqueurs ou plus sont élevés au-dessus d'un niveau de coupure ; et
la classification du patient comme endotype A / risque élevé ou autre qu'endotype A / risque élevé, une classification d'endotype A / risque élevé comprenant :
a) un niveau non élevé de JAK2 et un niveau non élevé de PRKCB ; ou
b) un niveau non élevé de JAK2,un niveau élevé de PRKCB, et un niveau non élevé de LYN ;
et une classification autre qu'endotype A / risque élevé comprenant :
c) un niveau non élevé de JAK2, un niveau élevé de PRKCB, et un niveau élevé de LYN ; ou
d) un niveau élevé de JAK2, un niveau non élevé de SOS2, et un niveau très élevé de LYN ; ou
e) des niveaux élevés de JAK2 et SOS2 ; ou
f) un niveau élevé de JAK2, un niveau non élevé de SOS2, et un niveau non très élevé de LYN.

2. Procédé selon la revendication 1, les niveaux d'expression de biomarqueurs étant déterminés par quantification d'ARNm, facultativement les niveaux d'expression de biomarqueurs étant déterminés par comptages normalisés d'ARNm et/ou par des valeurs de seuil de cycle (CT).

3. Procédé selon la revendication 1, dans lequel les biomarqueurs comprennent une paire de biomarqueurs choisie dans le groupe constitué par : JAK2 et LYN ; JAK2 et PRKCB ; JAK2 et SOS2 ; LYN et PRKCB ; LYN et SOS2 ; et PRKCB et SOS2 ; ou
dans lequel les biomarqueurs comprennent trois ou plus choisis dans le groupe constitué par JAK2, LYN, PRKCB et SOS2 ; ou
dans lequel les biomarqueurs comprennent un trio de biomarqueurs choisi dans le groupe constitué par : JAK2, LYN et PRKCB ; JAK2, LYN et SOS2 ; JAK2, PRKCB et SOS2 ; LYN, PRKCB et SOS2 ; ou
dans lequel les biomarqueurs comprennent tous parmi JAK2, LYN, PRKCB et SOS2.

4. Procédé selon l'une quelconque des revendications 1 à 3, dans lequel les niveaux de biomarqueurs sont déterminés par comptages normalisés d'ARNm, et dans lequel :
a) un niveau élevé de JAK2 correspond à un comptage d'ARNm JAK2 sérique supérieur à 1780 ;
b) un niveau élevé de PRKCB correspond à un comptage d'ARNm PRKCB sérique supérieur à 990 ;
c) un niveau élevé de SOS2 correspond à un comptage d'ARNm SOS2 sérique supérieur à 480 ;
d) un niveau élevé de LYN correspond à un comptage d'ARNm LYN sérique supérieur à 870 ; et
e) un niveau très élevé de LYN correspond à un comptage d'ARNm LYN sérique supérieur à 940 ;
comprenant facultativement l'application du schéma décisionnel de la Figure 1.

5. Procédé selon l'une quelconque des revendications 1 à 3, dans lequel les niveaux de biomarqueurs sont déterminés par comptages normalisés d'ARNm, et dans lequel :
a) un niveau élevé de JAK2 correspond à un comptage d'ARNm JAK2 sérique supérieur à 1784 ;
b) un niveau élevé de PRKCB correspond à un comptage d'ARNm PRKCB sérique supérieur à 986 ;
c) un niveau élevé de SOS2 correspond à un comptage d'ARNm SOS2 sérique supérieur à 480 ;
d) un niveau élevé de LYN correspond à un comptage d'ARNm LYN sérique supérieur à 873 ; et
e) un niveau très élevé de LYN correspond à un comptage d'ARNm LYN sérique supérieur à 938 ;
comprenant facultativement l'application du schéma décisionnel de la Figure 4.

6. Procédé selon l'une quelconque des revendications 1 à 5, dans lequel la détermination concernant le fait de savoir si les niveaux des deux biomarqueurs ou plus sont non élevés ou élevés au-dessus d'un niveau de coupure comprend l'application des données de niveau d'expression de biomarqueurs à un schéma décisionnel comprenant les deux biomarqueurs ou plus.

7. Procédé selon l'une quelconque des revendications 1 à 6, dans lequel une classification autre qu'endotype A / risque élevé comprend une classification d'endotype B / risque faible ou endotype C / risque modéré.

8. Procédé selon l'une quelconque des revendications 1 à 7, dans lequel la détermination concernant le fait de savoir si les niveaux des deux biomarqueurs ou plus sont non élevés ou élevés au-dessus d'un niveau de coupure est combinée avec une ou plusieurs données démographiques de patients et/ou caractéristiques cliniques et/ou résultats provenant d'autres tests ou signes de choc septique ; facultativement
dans lequel les données démographiques de patients et/ou caractéristiques cliniques et/ou résultats provenant d'autres tests ou signes de choc septique comprennent au moins l'un choisi dans le groupe constitué par l'organisme responsable de choc septique, la présence ou absence de maladie chronique, et/ou l'âge, le sexe, la race et/ou les comorbidités du patient.

9. Procédé selon l'une quelconque des revendications 1 à 8, dans lequel la détermination concernant le fait de savoir si les niveaux des deux biomarqueurs ou plus sont non élevés ou élevés au-dessus d'un niveau de coupure est combinée avec un ou plusieurs scores de risque supplémentaires basés sur la population ; facultativement le ou les scores de risque basés sur la population comprenant au moins l'un choisi dans le groupe constitué par risque de mortalité pédiatrique (PRISM), indice de mortalité pédiatrique (PIM) et/ou dysfonctionnement des organes logistiques pédiatriques (PELOD).

10. Procédé selon l'une quelconque des revendications 1 à 9, dans lequel l'échantillon a été obtenu au cours de la première heure de présentation de choc septique ; ou
dans lequel l'échantillon a été obtenu au cours des premières 48 heures de présentation de choc septique.

11. Procédé selon l'une quelconque des revendications 1 à 10, comprenant en outre l'identification d'un patient qui n'est pas endotype A / risque élevé et est un candidat pour un traitement comprenant un ou plusieurs corticostéroïdes, ou l'identification d'un patient qui est classé comme endotype A / risque élevé et est un candidat pour un traitement comprenant une ou plusieurs thérapies excluant un corticostéroïde ; facultativement
dans lequel le traitement pour le patient qui est classé comme endotype A / risque élevé comprend une ou plusieurs thérapies à haut risque ; facultativement
dans lequel le traitement pour le patient qui est endotype A / risque élevé comprend une ou plusieurs thérapies à haut risque choisies dans le groupe constitué par thérapie de renforcement immunitaire, oxygénation par membrane extracorporelle/respirateur artificiel, plasmaphérèse, cathétérisme de l'artère pulmonaire et/ou hémofiltration continue à haut volume ; facultativement
dans lequel la thérapie de renforcement immunitaire comprend GMCSF, interleukine-7, et/ou anti-PD-1.

12. Procédé selon la revendication 11, dans lequel un traitement comprenant un ou plusieurs corticostéroïdes est fourni à un patient qui n'est pas endotype A / risque élevé, ou un traitement comprenant une ou plusieurs thérapies excluant un corticostéroïde est fourni à un patient qui est classé comme endotype A / risque élevé, ce qui améliore une issue chez un patient pédiatrique en choc septique.

13. Procédé selon la revendication 11 ou 12, comprenant en outre :
dans un échantillon obtenu auprès du patient à un moment ultérieur, l'analyse de l'échantillon ultérieur pour déterminer les niveaux d'expression des deux biomarqueurs ou plus choisis dans le groupe constitué par JAK2, LYN, PRKCB et SOS2, et le fait de déterminer si les niveaux d'expression de deux biomarqueurs ou plus sont non élevés ou élevés au-dessus d'un niveau de coupure, ce qui détermine un changement à la classification d'endotype du patient ; et
le fait de déterminer que le traitement doit être maintenu si la classification d'endotype du patient n'a pas changé, ou que le traitement doit être changé si la classification d'endotype du patient a changé.

14. Utilisation d'une trousse, d'un test ou d'une matrice de diagnostic, comprenant une sonde d'hybridation rapporteuse, et une sonde d'hybridation de capture spécifique pour chacun parmi deux marqueurs d'ARNm ou plus choisis dans le groupe constitué par JAK2, LYN, PRKCB et SOS2, dans un procédé selon la revendication 1 ; facultativement
dans laquelle les marqueurs d'ARNm comprennent JAK2, LYN, PRKCB et SOS2.

15. Utilisation selon la revendication 14, dans laquelle la trousse, le test ou la matrice de diagnostic comprend en outre une cartouche de recueil pour immobilisation des sondes d'hybridation ; facultativement
dans lequel les sondes d'hybridation rapporteuse et de capture comprennent des éléments de signal et de code-barres, respectivement.
